(19) Europäisches Patentamt
European Patent Office
Office européen des brevets

(11) EP 0 695 746 A2

(12) **EUROPEAN PATENT APPLICATION**

(43) Date of publication:
**07.02.1996 Bulletin 1996/06**

(21) Application number: **95305336.0**

(22) Date of filing: **31.07.1995**

(51) Int Cl.6: **C07D 303/04**, C07D 409/04, A61K 31/335, A61K 31/38, A61K 31/34

(84) Designated Contracting States:
**BE CH DE DK ES FR GB IT LI NL SE**

(30) Priority: **01.08.1994 US 283732**

(71) Applicant: **PHARMA MAR, S.A.**
**E-28760 Tres Cantos, Madrid (ES)**

(72) Inventors:
- **De La Fuente, Jesus Angel**
  **Tres Cantos, E-28760 Madrid (ES)**
- **Marugan, Juan Jose**
  **Tres Cantos, E-28760 Madrid (ES)**
- **Cross, Sue S.**
  **Charlestown, Massachusetts 02129 (US)**

(74) Representative: **Ruffles, Graham Keith**
**London WC2A 3LS (GB)**

(54) **Antiviral compounds and pharmaceutical compositions**

(57) The present invention provides novel compounds and novel pharmaceutical compositions which possess antiviral activity, particularly against retroviruses. The compositions comprise a pharmaceutically acceptable carrier, diluent or excipient, and an effective antiviral amount, preferably anti-retroviral effective amount of a compound having the following generic formula:

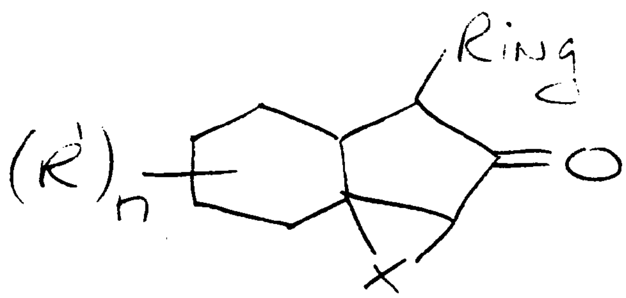

wherein X is selected from the group consisting of O and S; n is an integer of from 1 to 9, each $R^1$ is independently selected from the group consisting of hydrogen, $C_1$ to $C_4$ lower alkyl group (or others); and Ring is a $C_2$ to $C_6$ ring system containing up to three double bonds, and up to three heteroatoms selected from nitrogen, sulfur and/or oxygen.

EP 0 695 746 A2

## Description

### FIELD OF THE INVENTION

The present invention is directed to novel compounds and novel pharmaceutical compositions, which possess antiviral activity, particularly activity against retroviruses. The compounds of the present invention are also believed to be useful as insect antifeedants.

### BACKGROUND OF THE INVENTION

There are a number of viral diseases recognized as retroviral in origin in both humans and other animals. The most publicized of such disease among humans is that caused by the human immunodeficiency virus (HIV) as either AIDS or ARC. Other such diseases include hepatitis B and hepatitis delta. Among cats, retroviral diseases include those caused by the feline immunodeficiency virus (FIV) and the feline leukemia virus (FeLV). A number of other animal species also contract retroviral-caused infections, such as the Visna virus infections of ungulates.

Thus, as examples of retroviral infections which can be treated by administration of the compounds or compositions of the present invention include HIV, the causative agent in AIDS and HTLV-I (human T-lymphotropic virus type I), which causes leukemia and lymphoma. As an example of non-human animal retroviral infection treatable by administration of the compounds or compositions of the present invention, there may be mentioned FeLV (feline leukemia virus) which causes leukemia and immunodeficiency in cats. The compounds and compositions of the present invention also appear to be effective in the treatment of other retroviral infections, such as Herpes lesions, including chicken pox, EBV infection, and CMV infection.

### SUMMARY OF THE INVENTION

The compounds of the present invention all share the following basic structure:

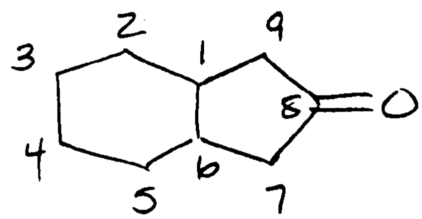

i.e., the bicyclo-[4.3.0]-nonan-8-one system.

Preferably, the compounds of the present invention are substituted 6,7-epoxy-bicyclo-[4.3.0]-nonan-8-ones, wherein the substituent groups are selected from the following; lower alkyl, preferably methyl, most preferably at position 1 and 5; and with a ring substituent (carbocyclic or heterocyclic) at position 9, as shown below:

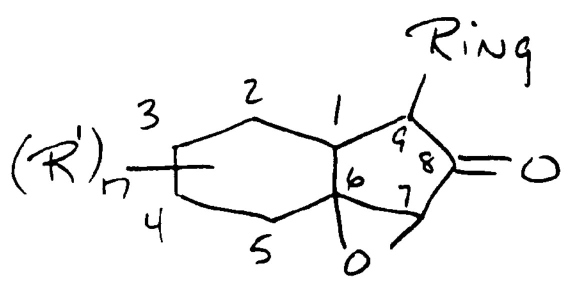

n = 1-9

The carbocyclic or heterocyclic ring systems suitable at position 9 in the structure shown above may be selected from the group consisting of substituted and unsubstituted rings including cyclopentadiene, pyrrole, furan, thiophene, benzene, pyridine, pyran, pyrone, naphthalene, quinoline, isoquinoline, quinolizine, acridine, phenanthridine, benzopyran, pyrazole, imidazole, isoxazole, oxazole, isothiazole, thiazole, pyridazine, pyrimidine, pyrazine, oxazine, thiazine, dioxane, purine, pteridine, triazine, sydnone, borepine, azepine, ozepine and thiepin.

Examples of such substituted 6,7-epoxy-bicyclo-[4.3.0]-nonan-8-ones are illustrated below:

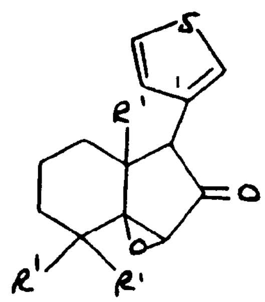
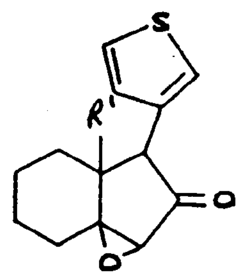
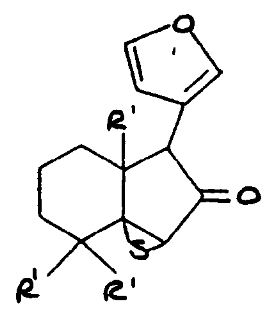

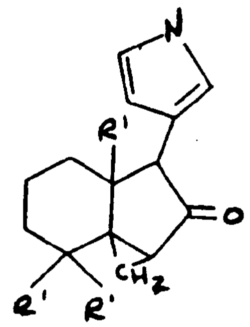
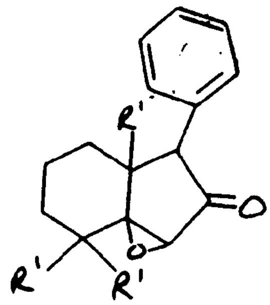
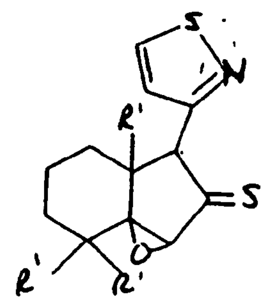

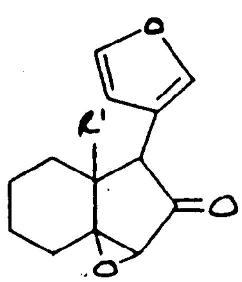
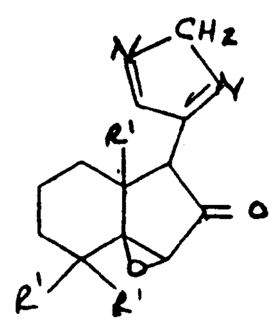
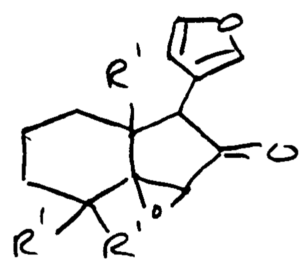

and the like.

As defined above, the compounds of the present invention (or substituents thereof) can be substituted or unsubstituted. Substituent groups useful herein include the following:

halogen, hydroxy, $CF_3$, $C_1$-$C_6$ acyl, $C_1$-$C_6$ alkyl, $C_2$-$C_6$ alkenyl, $C_2$-$C_6$ alkynyl, $C_1$-$C_6$ alkoxy, $C_6$-$C_{18}$ aryl, $C_2$-$C_6$ dialkoxymethyl, cyano, $C_3$-$C_6$ cycloalkyl, $C_3$-$C_6$ heterocycloalkyl, $C_3$-$C_{15}$ dialkylaminoalkyl, carboxy, $C_2$-$C_6$ carboxylic acid, carboxamido, $C_1$-$C_6$ haloalkyl, $C_1$-$C_6$ haloalkylthio, allyl, $C_7$-$C_{20}$ aralkyl, a $C_3$-$C_6$ heterocycloalkyl ring fused to a benzene ring, $C_1$-$C_6$ alkylthio, $C_1$-$C_6$ alkylsulfonyl, $C_1$-$C_6$ haloalkylsulfonyl, $C_1$-$C_6$ alkylsulfinyl, $C_1$-$C_6$ haloalkylsulfinyl, arylthio, $C_1$-$C_6$ haloalkoxy, amino, $C_1$-$C_6$ alkylamino, $C_2$-$C_{15}$ dialkylamino, hydroxy, carbamoyl, $C_1$-$C_6$ N-alkylcarbamoyl, $C_2$-$C_{15}$ N,N-dialkylcarbamoyl, nitro, $C_2$-$C_{15}$ dialkylsulfamoyl, and the like.

Typical $C_1$-$C_6$ alkyl groups include methyl, ethyl, n-propyl, i-propyl, n-butyl, t-butyl, i-butyl, pentyl and hexyl groups.

Typical $C_{3-8}$ cycloalkyl groups include cyclopropyl, cyclobutyl, cyclopentyl, cyclohexyl, cycloheptyl, and cyclooctyl groups.

Typical $C_2$-$C_6$ carboxylic acyl groups include acetyl, propanoyl, i-propanoyl, butanoyl, s-butanoyl, pentanoyl and hexanoyl groups.

Typical aryl groups include phenyl, naphthyl, phenanthryl, anthracyl and fluorene groups.

Typical aryl-substituted carboxylic acid groups include the above-mentioned carboxylic acyl groups substituted by one or more aryl groups, e.g., diphenylacetoxy and fluorenecarboxy groups.

Typical alkaryl groups include the above-listed aryl groups substituted by one or more $C_1$-$C_6$ alkyl groups.

Typical aralkyl groups include a $C_1$-$C_6$ alkyl group substituted by one of the above-listed aryl groups, e.g., phenethyl, phenylpropyl, phenylbutyl, phenylpentyl and phenylhexyl groups as well as the branched chain isomers thereof.

Typical $C_1$-$C_6$ alkoxycarbonyl groups include carbonyl substituted by methoxy, ethoxy, propanoxy, i-propanoxy, n-butanoxy, t-butanoxy, i-butanoxy, pentanoxy, and hexanoxy groups.

Typical aralkyl groups include the above-listed $C_1$-$C_6$ alkyl groups substituted by phenyl, naphthyl, phenanthryl, and anthracyl groups.

Typical $C_2$-$C_6$ alkenyl groups include vinyl, allyl, 2-butenyl, 2-pentenyl, and 2-hexenyl groups.

Typical $C_2$-$C_6$ alkynyl groups include acetynyl and propargyl groups.

Typical halo groups include fluorine, chlorine, bromine and iodine.

Typical aroyl groups include carbonyl substituted by phenyl, naphthyl, phenanthryl, and anthracyl groups.

Typical aralkanoyl groups include carbonyl substituted by the above-listed aralkyl groups.

Typical aralkoxy groups include the above listed $C_1$-$C_6$ alkoxy groups substituted by phenyl, naphthyl, phenanthyl, and anthracyl groups.

Typical substituted aryl groups include the above-listed aryl groups substituted by halo, hydroxy, $C_1$-$C_6$ alkoxy, amino, and the like.

Typical heteroaryl groups include furyl, thienyl, pyrrolyl, thiazolyl, pyridyl, pyrimidinyl, pyrizinyl, oxazolyl and phthalimido groups which may be fused to a benzene ring.

Typical substituted heteroaryl groups include the above-listed heteroaryl groups substituted by halo, $C_1$-$C_6$ alkyl and the like.

Typical $C_5$-$C_6$ heterocycloalkyl groups include tetrahydrofuranyl, tetrahydropyranyl, piperidinyl, piperazinyl, morpholino and pyrrolidinyl groups.

The substituted bicyclo-[4.3.0]-nonan-8-one ring system common to all of the above depicted compounds has at least one asymmetric carbon atom and as such, can exist as one or more pairs of nonidentical, mirror image molecules, called enantiomers. At the molecular level, one can distinguish the chirality of asymmetric molecules by observation of physical property differences, e.g., measurement of optical activity using dissymmetric, polarized light, in either a positive (+) or negative (-) direction. A mixture of two enantiomers (usually called a racemic mixture and commonly designated as (±)) will not cause light rotation, as it will usually consist of a 1:1 mixture of both the (+) rotational isomer and the (-) rotational isomer. Other physical properties (e.g., melting point, spectra, etc.) will not distinguish between enantiomeric forms, since these properties do not possess intrinsic asymmetry.

In view thereof, it has been discovered that different enantiomers of compounds of the present invention have surprisingly better activity against retroviruses *in vitro*. It is further believed that this unexpected activity will likewise be found *in vivo*.

Preferred compounds of the present invention have the following formula:

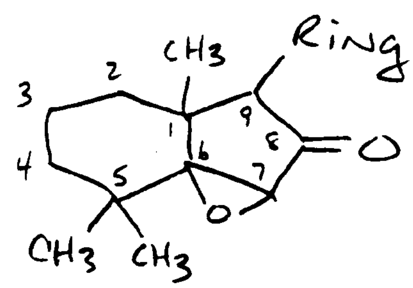

wherein Ring is selected from substituted and unsubstituted phenyl and thiophene.

As discussed in greater detail below, the formula provided above does not provide any specific information regarding the various conformations possible for the substituent groups present. The present inventors have isolated the following specific compounds, which are especially preferred herein:

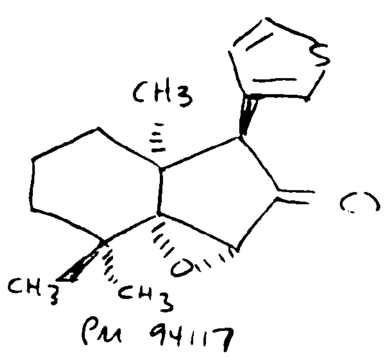

PM 94117

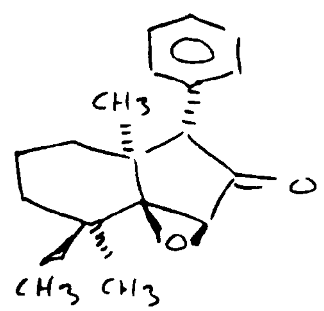

PM 94118 (±)

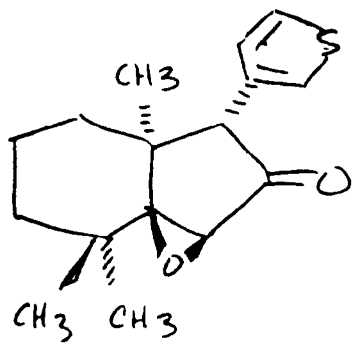

SU 0096 (±)

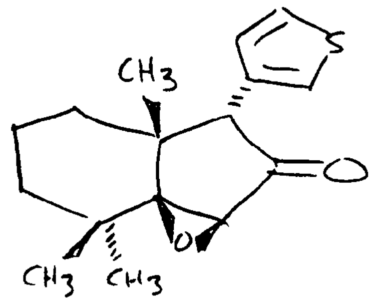

PM 94116

As illustrated above, the epoxide ring (or its N or S equivalent) in active compounds of the present invention can have two different conformations, relative to the plane of symmetry for the compound. If one assumes that the ketone group at position 8 defines this plane, the epoxide ring can be either above the plane (as in PM 94116 and SU 0096), or below the plane (as in PM 94117).

Similarly, if one or more methyl groups ($CH_3$) are attached to the basic bicyclo-[4.3.0]-nonan-8-one ring system, particularly at position 1, the methyl group(s) may, as illustrated above, present several conformational possibilities.

Finally, the carbocyclic or heterocyclic ring attached at position 9 can also have two possible conformations, above or below the plane of symmetry.

Clearly, if three asymmetric groups are present, e.g., a $CH_3$ at position 1, an epoxide at position 6-7 and carbocyclic or heterocyclic ring at position 9; there are at least eight ($2^3$) possible combinations. To simplify this, the compounds of the present invention, for purposes of the claims, will be depicted as flat structures. However, it is intended that all possible isomers, geometric, and enantiomeric, are included within the scope thereof. In specific instances, the actual conformation may be claimed as well, particularly when the spatial geometry provides enhanced antiviral activity.

The following racemic compound is not being claimed herein as a new compound. The racemic mixture, as well as related structural analogs, synthetic intermediates and the synthesis thereof, are described in Mateos et al., *J. Org. Chem.*, **1990**, *55*, 1349-1354, the disclosure of which is hereby incorporated herein by reference.

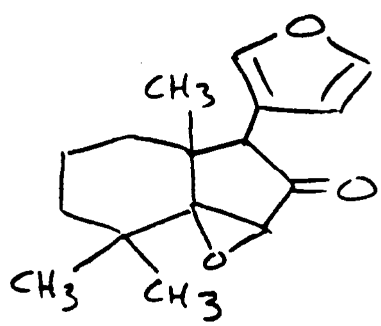

Formula I

Prior to the present invention, the individual enantiomers of Formula I (shown below as Formula A and Formula B) had not been isolated. The only previously reported utility for the racemic compound of Formula I was as an insect antifeedant (Mateos et al., *supra*). Thus, these compounds are being claimed herein as an active ingredient in an antiviral, preferably an anti-retroviral, pharmaceutical composition. The insect antifeedant activity of the Mateos et al. compound is likely to be possessed by the new compounds of the present invention.

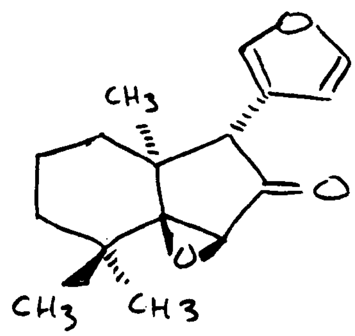

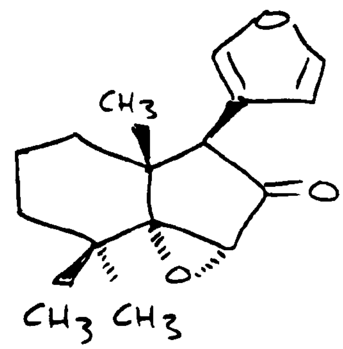

A (PM-92131(+)) B (PM-92131(-))

As described above, the present invention is directed both the novel antiviral compounds, as well as to pharmaceutical compositions which include compounds exhibiting antiviral activity. Thus, the pharmaceutical compositions of the present invention are directed to compositions comprising a pharmaceutically acceptable carrier, diluent or excipient, and an effective antiviral amount, particularly an effective anti-retroviral amount of a compound having the following generic formula:

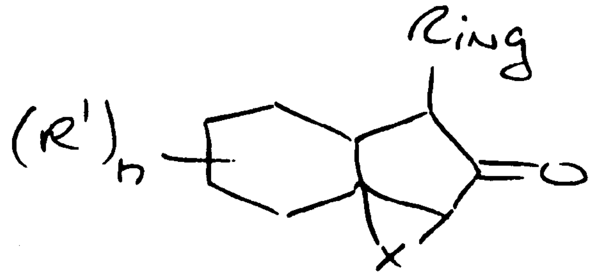

wherein X is selected from the group consisting of O and S; n is an integer of 1-9; each $R^1$ is preferably either H or a $C_1$ to $C_4$ lower alkyl group; and Ring is a $C_3$ to $C_6$ carbocyclic or $C_2$ to $C_5$ heterocyclic ring system with up to three heteroatoms selected from nitrogen, sulfur and/or oxygen, and wherein either ring system may containing up to three carbon-carbon double bonds.

In one preferred embodiment of the present invention, the compositions of the present invention include, as the active antiviral ingredient, compounds having the following sub-generic formula:

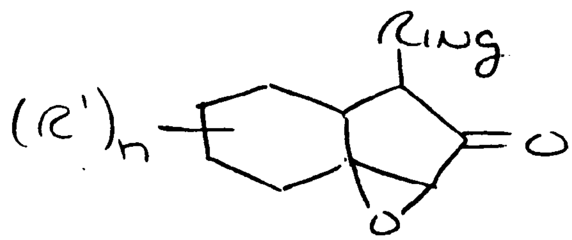

In another preferred embodiment of the present invention, the compositions of the present invention include, as the active antiviral ingredient, compounds having the following sub-generic formula:

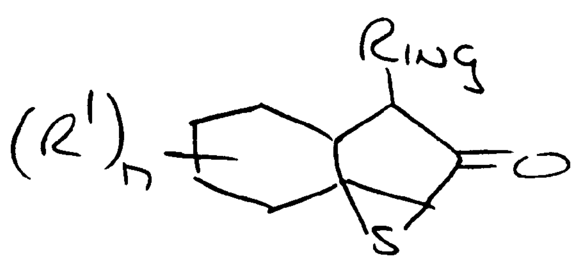

In each of the two sub-generic formulas shown above, the following ring systems are especially preferred:

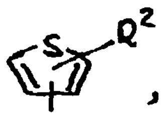 , 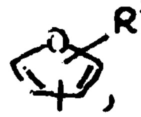 , 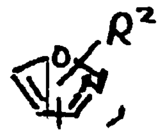 ,

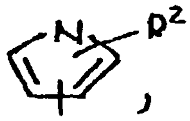 , 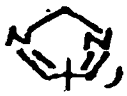 , 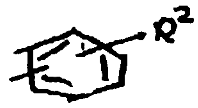

Examples of compounds falling within the generic formula shown above include the following (wherein $R^2$ is defined as above for $R^1$):

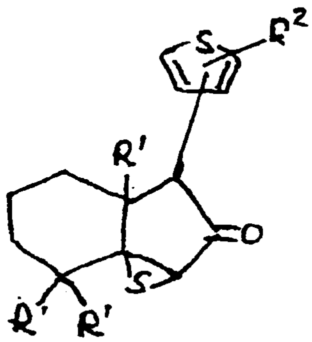
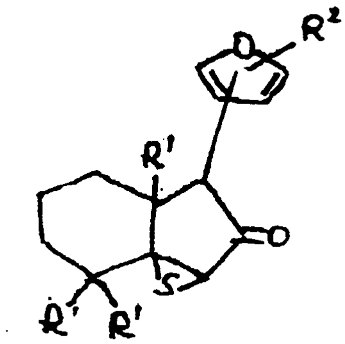
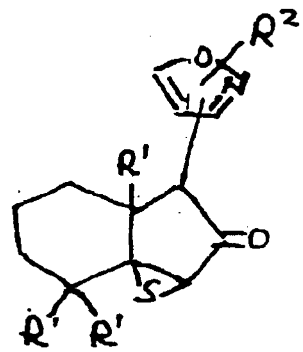

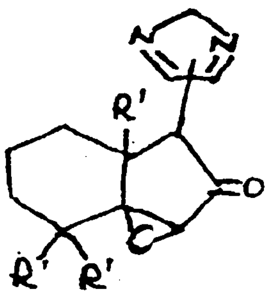
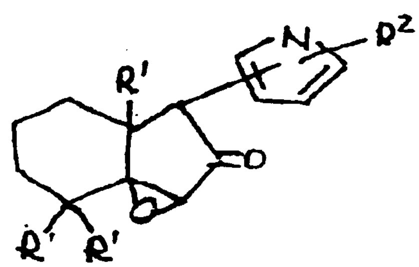
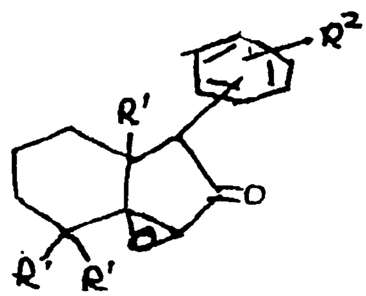

If desired, the bicyclo-[4.3.0]-nonan-8-one rings system can be further fused to one or more additional carbocyclic rings, providing the following structures:

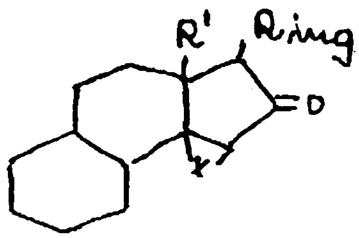
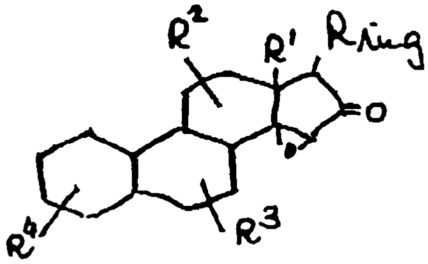

and the like (wherein $R^3$ and $R^4$ are independently defined as above for $R^1$).

**DETAILED DESCRIPTION OF THE PREFERRED EMBODIMENTS**

The compounds and compositions of the present invention are useful in medical therapy particularly for the treatment of viral infections, especially retroviral infections and hepatitis B viral infections in, e.g., humans. Examples of retroviral infections which may be treated in accordance with the present invention include human retroviral infections such as HIV-I, HIV-2 and Human T-cell Lymphotropic Virus (HLTV) e.g., HTLV-I or HTLV-II infections.

The compounds and compositions of the present invention are also useful for the treatment of clinical conditions

associated with retroviral infections, for example, AIDS, Kaposi's sarcoma, thrombocytopenic purpura, AIDS-related complex (ARC), progressive generalized lymphadenopathy (PGL), and patients carrying HIV-antibodies or those who are seropositive to the HIV virus.

The racemic compound PM-92131(±), as well as synthetic intermediates and the synthesis thereof, are described in Mateos et al., *J. Org. Chem.*, 55, 1349-1354 (1990). Prior to the present invention, the individual enantiomers (PM 92131(+) and PM-92131(-)) had not been isolated.

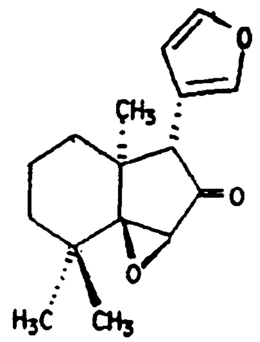

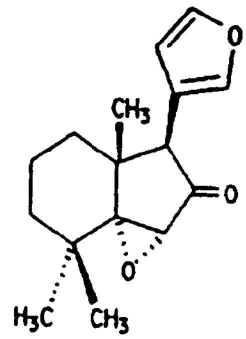

PM-92131(+) PM-92131(-)

The isolation of each enantiomer was accomplished by application of synthetic methods, such as those shown below:

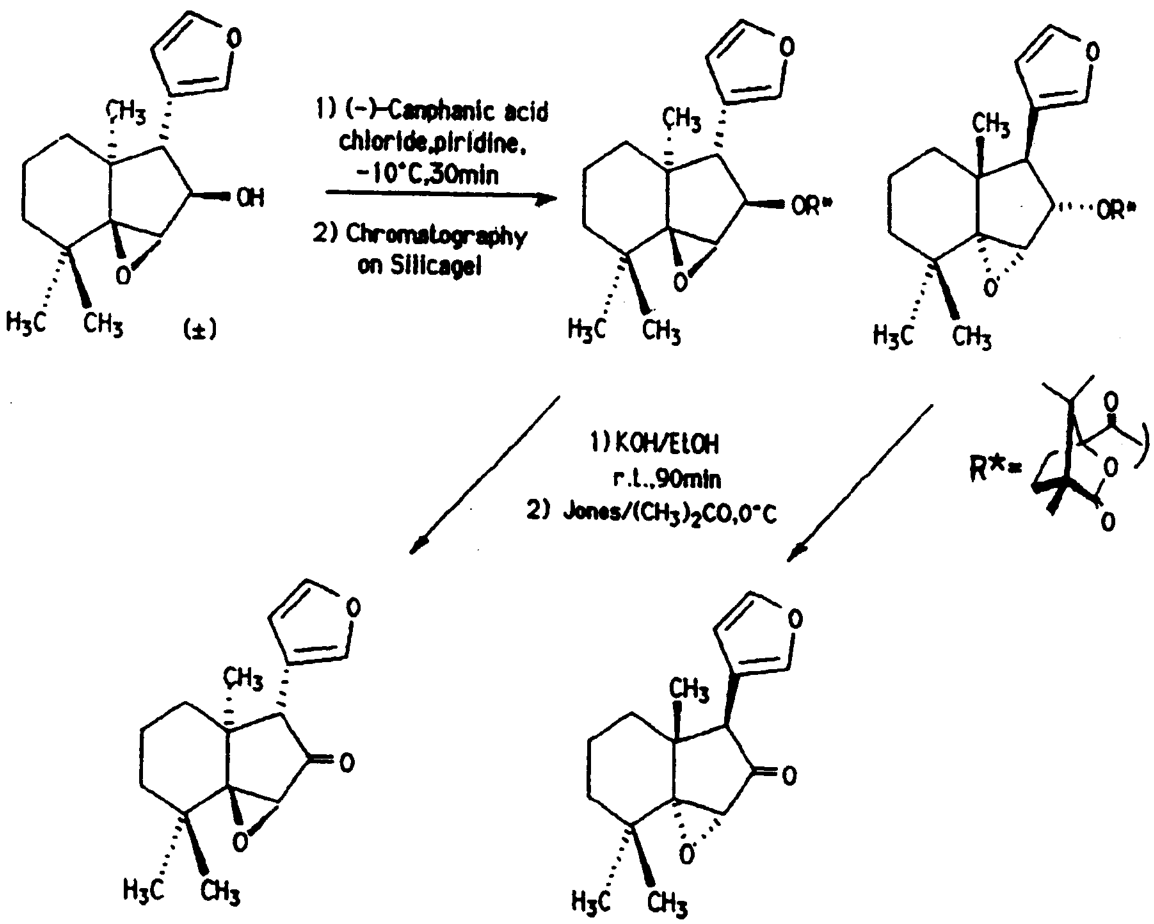


**PM-92131(+)** **PM-92131(-)**

The enantiomers PM-92131(+) and PM-93131(-) are clearly identified by their optical rotation properties:

PM-92131(-): $[\alpha]_D^{20}$ = -29.1° (c = 2.82, $CHCl_3$)

PM-92131(+): $[\alpha]_D^{20}$ = +28.7° (c = 3.21, $CHCl_3$)

The absolute configuration of each enantiomer has been determined by X-ray crystallography of the most active one.

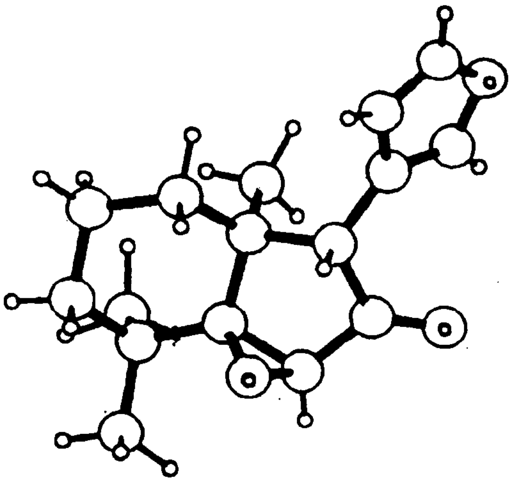

PM-92131(+)

New active compounds obtained by the present inventors include:

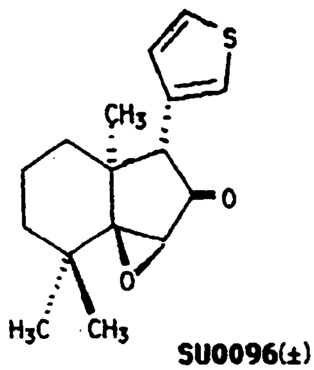

SU0096(±)

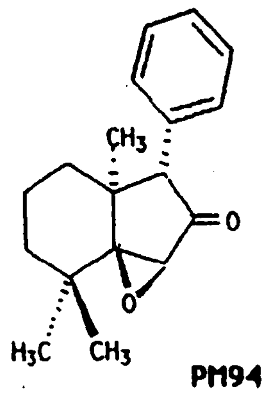

PM94118(±)

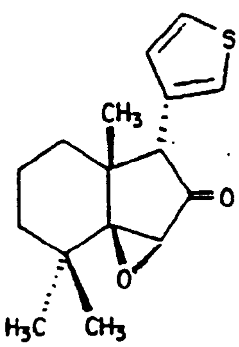

PM94116+PM94117
1:1 ratio

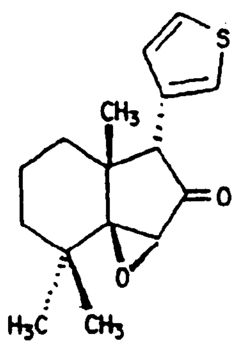

PM94116

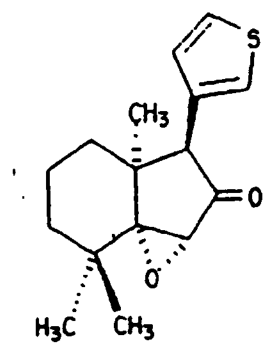

PM94117

SU-0096(±) and PM-94118(±) were synthesized by the same synthetic pathway described in Mateos et al., in which the nitro olefin therein is changed by SU-0091 and SU-0105 respectively

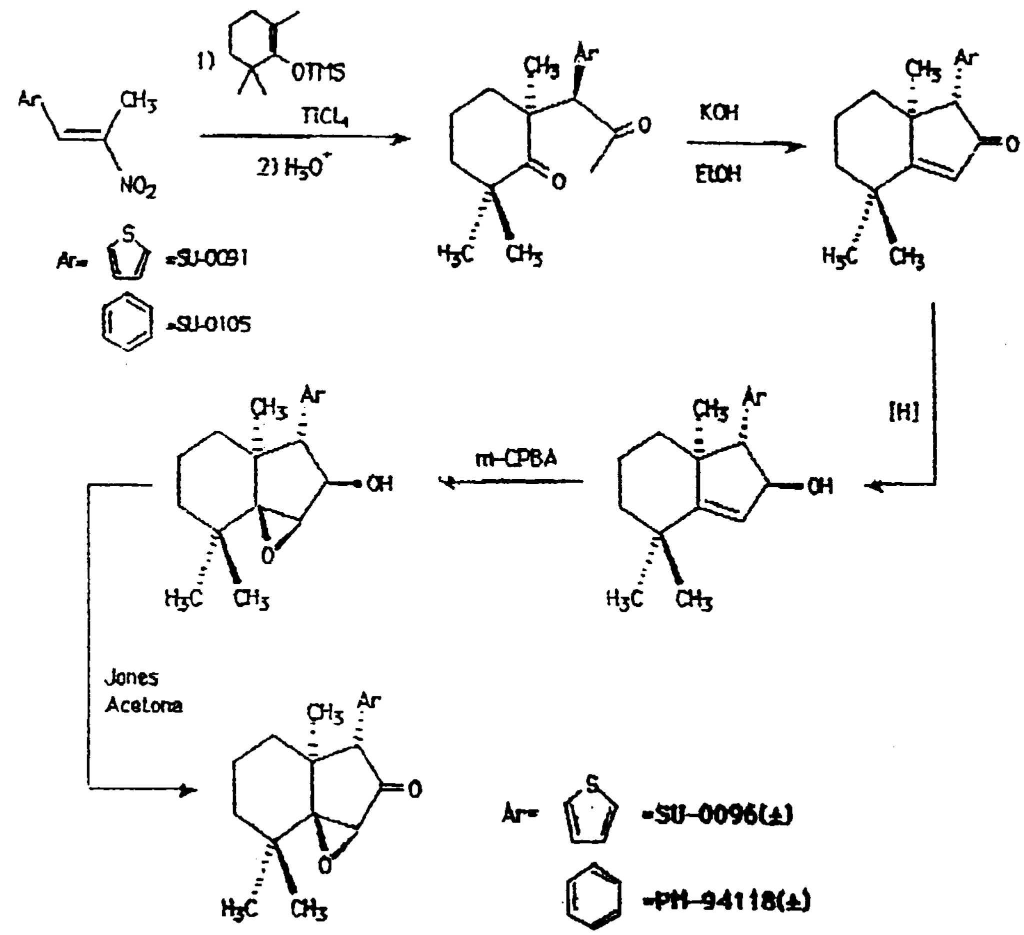


SU-0096(±) having the following characteristics: white solid, m.p.: 107.2 °C; IR ($CHCl_3$) 1742, 1464, 1392 and 1137 $cm^{-1}$; $^1H$ NMR (300 MHz, $CDCl_3$) $\delta$ 7.25 (1H, dd, J=4.9, 3.0 Hz), 7.15 (1H, m) 6.86 (1H, dd, J=4.9, 1.2 Hz), 4.06 (1H, s), 3.45 (1H, s), 1.95 - 1.55 (6H, m), 1.22 (3H, s), 0.84 (3H, s), 0.82 (3H, S); $^{13}C$ NMR (300 MHz, $CDCl_3$) $\delta$ 210.03 (s), 133.19 (s), 128.79 (d), 124.53 (d), 123.92 (d), 74.47 (s), 57.54 (d), 54.47 (d), 44.02 (s), 38.90 (t), 33.70 (s), 33.37 (t), 26,96 (q), 25.17 (q), 18.72 (t) and 18.63 (q).

PM-94118(±), having the following characteristics: white solid, m.p.: 98.3°C; IR 2949, 1749, 1466, 1450, 1141, 892, 824, 755 and 702 $cm^{-1}$; $^1H$ NMR (300 MHz, $CDCl_3$) $\delta$ 7.30 (3H, m), 7.09 (2H, m), 3.97 (1H, s), 3.48 (1H, s), 1.95-1.55 (6H, m), 1.24 (3H, s) and 0.86 (6H, s); $^{13}C$ NMR (300 MHz, $CDCl_3$) $\delta$ 210.02 (s), 133.17 (s), 130.79 (d)x2, 127.89 (d) x2, 126.98 (d), 74.27 (s), 58.83 (d), 57.78 (d), 44.39 (s), 39.04 (t), 33.83 (s), 33.39 (t), 27.13 (q), 25.30 (q), 20.17 (t) and 18.67 (q).

To obtain the racemic mixture PM-94116/PM-94117, SU-0091 was used as nitro olefin and some modifications were introduced in the general synthetic pathway, as depicted below.

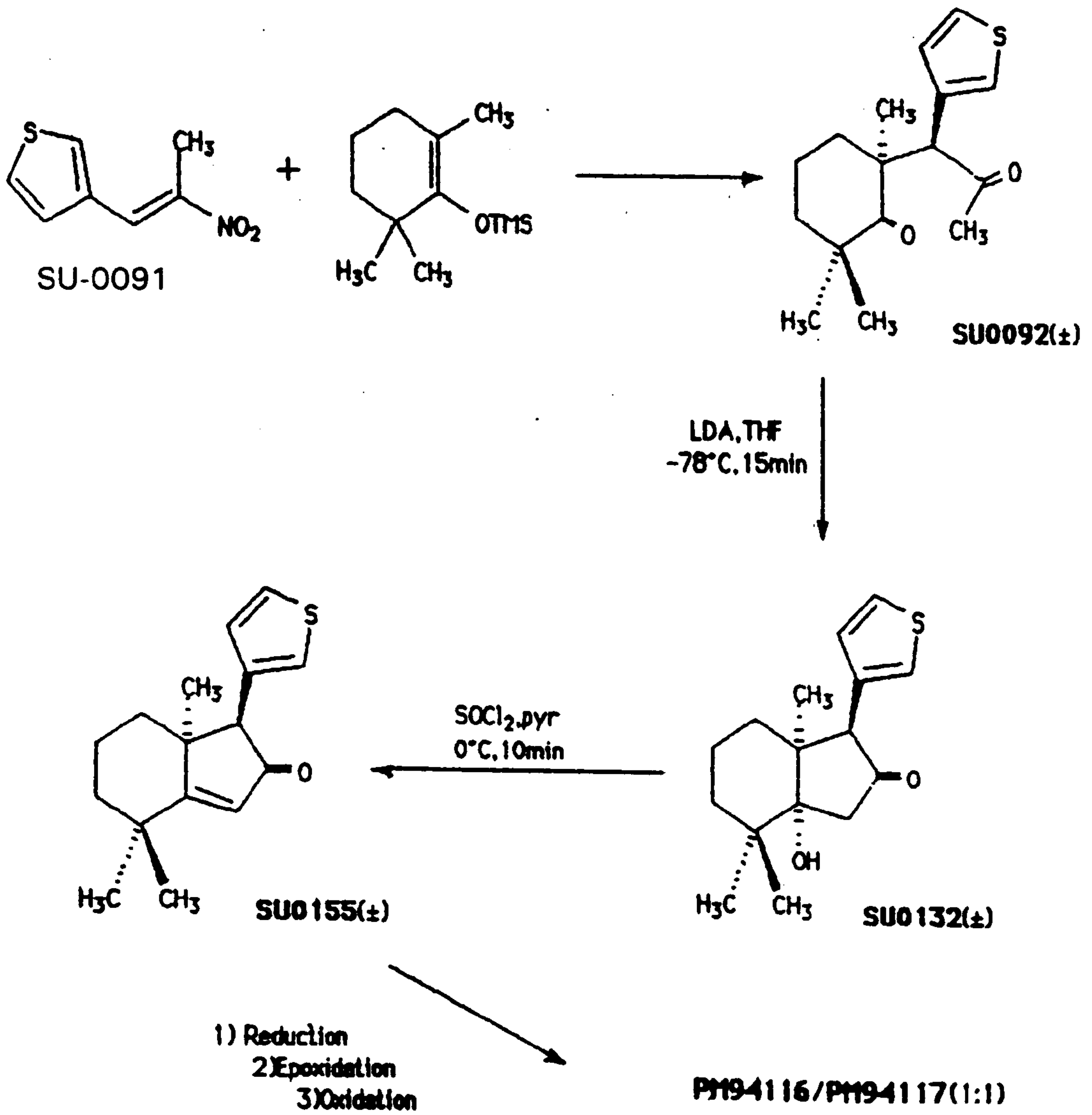


SU-0091

SU0092(±)

SU0132(±)

SU0155(±)

PM94116/PM94117(1:1)

PM-94116/PM-94117, having the following characteristics: IR: 1733, 1467, 1376 and 1176 cm$^{-1}$; $^{1}$H NMR (300 MHz, $CDCl_3$) δ 7.29 (1H, dd, J=5.0, 3.0 Hz), 7.03 (1H, dd, J=3.0, 1.3 Hz), 6.78 (1H, dd, J=5.0, 1.3 Hz), 3.81 (1H, s) 3.43 (1H, s), 1.36 (3H, s), 1.25 (3H, s) and 0.84 (3H, s); $^{13}$C NMR (300 MHz, $CDCl_3$) δ 210.02 (s), 132.319 (s), 129.03 (d), 124.77 (d), 124.62 (d), 75.59 (s), 60.59 (d), 55.55 (d), 42.99 (s), 40.31 (t), 35.19 (t), 33.50 (s), 27.29 (q), 24.49 (q), 20.56 (q) and 17.94 (t).

The enantiomers PM-94116 and PM-94117 were separated using the same approach described to separate PM-92131(+) and PM-92131(-).

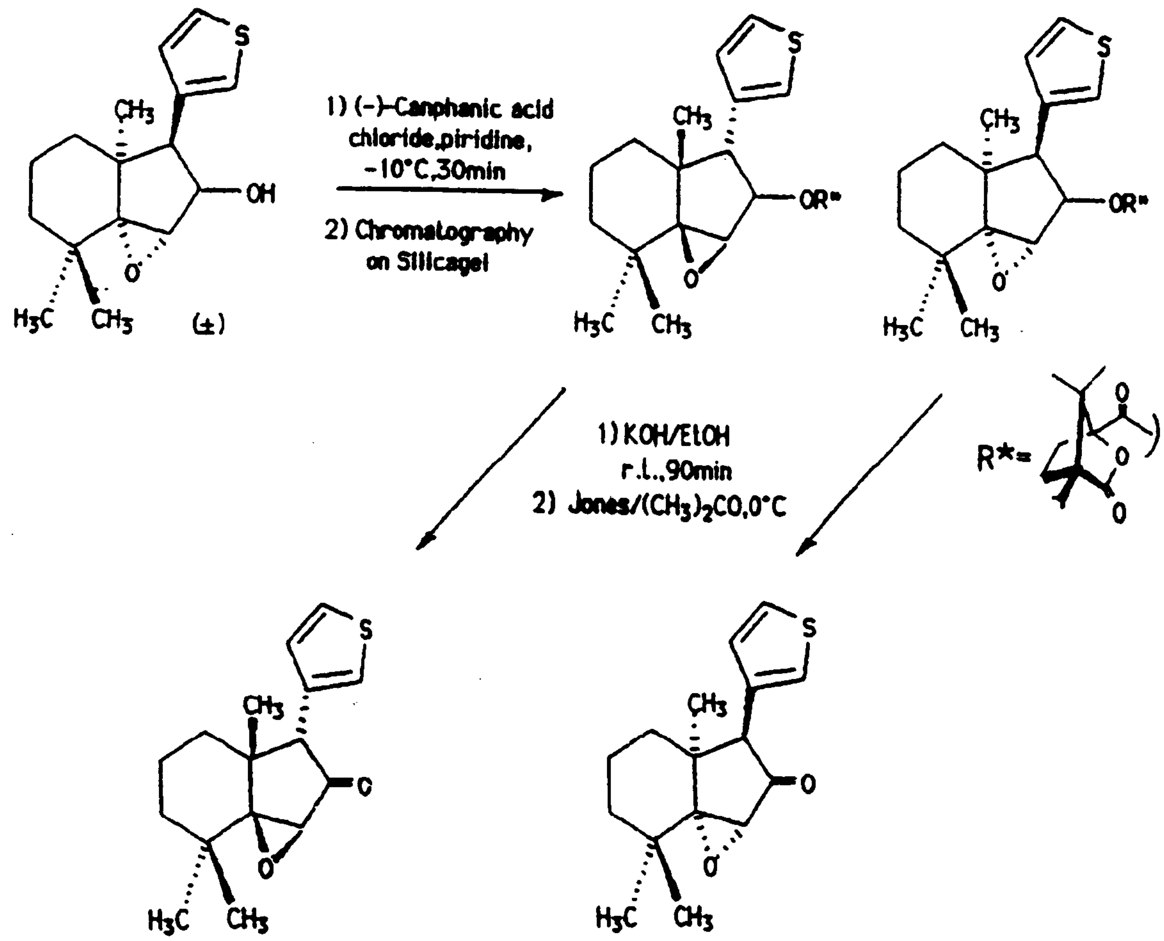


PM-94116 PM-94117

The enantiomers PM-94116 and PM-94117 are clearly identified by their optical rotational properties:

PM-94116: $[\alpha]_D^{20}$ = + 73.67° (c = 0.13, $CHCl_3$)

PM-94117: $[\alpha]_D^{20}$ = - 70.57° (c = 0.15, $CHCl_3$)

The absolute configuration of each enantiomer has been determined by X-ray crystallography of the most active one.

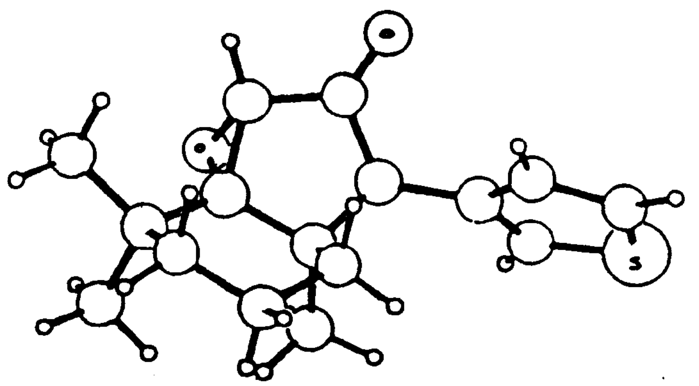

PM-94116

The preferred antiviral compounds or compositions of the present invention may be employed in combination with other therapeutic agents for the treatment of the above defined infections or conditions. Examples of such further therapeutic agents include agents that will be effective for the treatment of viral infections or associated conditions such as 3'-azido-3'-deoxythymidine (zidovudine), 2',3'-dideoxynucleosides such as 2',3'-dideoxycytidine, 2',3'-dideoxyadenoside and 2',3'-dideoxyinosine, acylic nucleosides (e.g., acyclovir), interferons such as α-interferons, β-interferons, and γ-interferons, renal excretion inhibitors such as probenicid, nucleoside transport inhibitors such as dipyridamole, dilazep, mio-, lido- or soluflazine, or hexobendine, immunomodulators such as interleukin II and granulocyte macrophage colony

stimulating factors, soluble $CD_4$ or genetically engineered derivatives thereof, and phosphonoformic acid. The component compounds of such combination therapy may be administered simultaneously, in either separate or combined formulations, or at different times, e.g., sequentially such that a combined effect is achieved.

The antiviral compounds and compositions according to the present invention may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the active ingredients.

In general a suitable dose for each of the above-mentioned conditions (e.g., AIDS) will be in the range of from about 5 to 500 mg per kilogram body weight of the recipient (e.g., a human) per day, preferably in the range of from about 10 to 200 mg per kilogram body weight per day and most preferably in the range of from about 20 to 100 mg per kilogram body weight per day. The desired dose is preferably presented as two, three, four, five, six or more sub-doses administered at appropriate time intervals throughout the day. These sub-doses may be administered in unit dosage form, for example, containing from about 2.5 to 250 mg, preferably from about 7.5 to 100 mg, and most preferably from about 10 to 40 mg of active ingredient per unit dosage form.

Ideally, the active ingredient should be administered to achieve peak plasma concentrations of the active ingredient of from about 1 to 100 μM, preferably from about 5 to 8 μM, most preferably about 7.5 to 50 μM. This may be achieved, for example, by the intravenous injection of a 0.1 to 5% solution of the active ingredient, optionally in saline, or orally administered as a bolus containing about 1 to about 100 mg/kg of the active ingredient. Desirable blood levels may be maintained by continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation. The formulations of the present invention comprise the active ingredient, as defined above, together with at least one pharmaceutically acceptable carrier, diluent or excipient. Preferred formulations include those adapted for oral, rectal, nasal, topical (including buccal and sublingual), vaginal or parenteral (including subcutaneous, intramuscular, intravenous and intradermal) administration. The formulations may conveniently be presented in unit dosage form and may be prepared by methods well known in the art of pharmacy. Such methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then if necessary shaping the product.

Formulations of the present invention adapted for oral administration may be presented as discrete units such as capsules or tables each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder (e.g., povidone, gelatin, hydroxypropylmethyl cellulose), lubricant, inert diluent, preservative, disintegrant (e.g., sodium starch glycolate, cross-linked providone, cross-linked sodium carboxymethyl cellulose) surface-active or dispersing agent.

Molded tablets may be made by molding in a suitable machine a mixture of the powdered compound moistened with an inert liquid diluent. The tablets may optionally be coated or scored and may be formulated so as to provide slow or controlled release of the active ingredient therein using, for example, hydroxypropyl-methyl cellulose in varying proportions to provide the desired release profile. Tablets may optionally be provided with an enteric coating, to provide release in parts of the gut other than the stomach.

Formulation adapted for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations adapted for rectal administration may be presented as a suppository with a suitable base comprising for example cocoa butter or a salicylate.

Formulations adapted for vaginal administration may be presented as pessaries, tampons, creams, gels, pastes, foams or spray formulations containing in addition to the active ingredient such carriers as are known in the art to be appropriate.

Formulations adapted for parenteral administration include aqueous and non-aqueous isotonic sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be present in unit-dose or multidose sealed containers, for example, ampules and vials, and may be stored in a freeze-dried (lyophilized) condition requiring only the addition of the sterile liquid carrier, for example water for injections, immediately, prior to use. Extemporaneous injection solutions and suspensions may be prepared from sterile powders, granules and tablets of the kind previously described.

Preferred unit dosage formulations as those containing a daily dose or unit, daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example, those suitable for oral administration may include such further agents as sweeteners, thickeners and flavoring agent.

The present invention will be further illustrated with reference to the following examples which aid in the understanding of the present invention, but which are not to be construed as limitations thereof. All percentages reported herein, unless otherwise specified, are percent by weight. All temperatures are expressed in degrees Celsius.

## EXAMPLES - ANTIVIRAL ACTIVITY

### A. Virus stock

The virus stock for testing crude extracts was HIV-1 RF (HTLV-$III_{RF}$/H9), a strain provided by Dr. Owen Weislow, Program Resources, Inc., Frederick, MD. This strain was selected for its cytopathic and lytic effects on CEM-SS human lymphoid cells.

For the development of a good antiviral assay test both the storage and preparation of the viral stocks are important. A high titered viral stock stored at ultralow temperatures provides a standardized reagent that can be used over a period of months for numerous assays, and the results can be compared, and replicates performed on different test days are meaningful.

### A.1. Preparation of virus stock

For the antiviral assay used to test the compounds discussed in this patent, HIV-1 RF infectious viral stocks were prepared in H9 cells. The original H9 cells were also provided by Dr. Owen Weislow. The H9 cells were a single cell clone derived from a specific HUT 78 cell line. HUT 78 is a human cutaneous T-cell lymphoma derived from the peripheral blood of a patient with Sezary syndrome. The H9 cells were cloned from HUT 78 by Popovic et al., "Detection, Isolation, and Continuous Production of Cytopathic Retroviruses (HTLV-III) from Patients with AIDS and pre-AIDS," *Science*, **224**: 497 (1984). The cells grow in suspension and are less sensitive to cell lysis by HIV than some other cell lines. The cell line is permissive for HIV growth and high viral yields can be obtained.

### A.2. Procedure for preparation of HIV viral stocks

H9 cells were maintained in RPMI-1640 media supplemented with 10% fetal bovine serum, 2% L-glutamine (200 mM), and 50 µg/ml gentamicin. The cultures were maintained in a logarithmic state of growth by subculturing them at a cell concentration of 2.5 x $10^5$ cells/ml and allowing them to divide until the cell concentration reaches 1 x $10^6$ per ml, at which time the cells are again subdivided into new culture medium.

For infection of H9 cells with HIV-1, 50-ml volumes of cells at a concentration of 5 x $10^5$ H9 cells were treated with polybrene (hexadimethrine bromide, Sigma catalog, cat. #H 9268) at 1 µg/ml for 30 minutes at 37°C. At the end of the incubation period, cells were centrifuged at 800 rpm for 10 minutes and supernatant fluid was discarded. Cell pellets were infected with HIV-1 virus at an effective M.O.I. (multiplicity of infection) of approximately 0.1. Cell pellet and virus were incubated at 37°C for 1 hour with occasional shaking. At the end of the incubation period 22.5 x $10^6$ cells infected with HIV-1 were resuspended in 75 ml growth medium. Cells were cultured for two days at 37°C. After 48 hours cells were removed by centrifugation at 1000 rpm for 10 minutes and the supernatant was distributed into 1-ml vials suitable for storage in a liquid nitrogen storage facility.

Some additional steps to the above procedure worked to increase the viral titers of the stock virus and eliminate noninfectious virus. One was the addition of uninfected H9 cells to the pool 24 and 48 hours after infection and two, supernatant fluids from the infected cells that were frozen, thawed and centrifuged to eliminate cell debris were added to the pool.

### A.3. Titration of HIV-1 virus stock for antiviral assays

To determine the HIV-1 viral titers the quantitative infectivity syncytium-forming assay described by P.L. Nara and P.J. Fischinger, "Quantitative Infectivity Assay for HIV-1 and -2," *Nature*, **332**: 469 (1988)) was used. In this assay a single infectious unit of virus infects a single cell and initiates a focal cell change such as syncytium formation. Because a single infectious unit causes a single response, a linear relationship exists between the number of cytopathic effects

caused by viral infection and the first order of virus concentration. This quantitative assay can be used to determine the titers of the HIV-1 in viral stocks, neutralization tests, and antiviral assays.

The quantitative infectivity syncytium-forming assay is applied for the direct quantitation of fusigenic virus-infected cells. CEM-SS cells were used for the syncytium-forming assay. CEM-SS cell are human T4-lymphoblastoid cells of a cell line initially derived by G.E. Foley, et al., "Continuous Culture to Human Lymphoblasts from Peripheral Blood of a Child with Acute Leukemia," *Cancer*, **18**: 522 (1965) and biologically cloned by Nara et al., *supra*. The cells for this assay were provided by Dr. Nara.

CEM-SS cells were maintained in a growth medium of RPMI 1640, 10% fetal bovine serum, 1% L-glutamine (200 mM) and 50 µg/ml of gentamicin. CEM-SS cells grow in suspension. The cells have been cloned for both poly-L-lysine-induced adherence to microtiter plates and virus-induced syncytial and fusogenic sensitivity following infection with either cell-free or cell-associated HIV-1 and HIV-2. It has been determined that the cells are negative for virus including human retroviruses as determined by electron microscopy and reverse transcriptase analysis.

**A.4. Outline of syncytium-forming assay for evaluation of viral titers.**

**A.4.1.** Use 96-wells flat bottom tissue culture plates and score the bottoms of all wells into 4 quadrants.

**A.4.2.** Add 50 µl/well of diluted poly-L-lysine.
Prepare stock poly-L-lysine (Poly-L-lysine hydrobromide, Sigma catalog, cat. # P-1399) at 5 mg/ml and store at -20°C for future use. For the assay dilute the stock poly-L-lysine 1:100 using phosphate buffered saline (PBS).

**A.4.3.** Incubate plates at room temperature for no less than 30 minutes.

**A.4.4.** Remove poly-L-lysine from wells.

**A.4.5.** Wash the plates with 100 µl/well of Dulbecco's PBS and remove the excess PBS.

**A.4.6.** Dilute CEM-SS cells in a logarithmic state of growth to 0.5 x $10^6$ cells per ml in a serum free medium and add 1 µg/ml polybrene. Incubate the cell suspension at 37°C for 60 minutes. Centrifuge at 800 rpm for 10 minutes and decant the supernatant fluid.

**A.4.7.** Dilute the treated CEM-SS cell suspension to 5.0 x $10^5$ cells. Add 100 µl to the test wells so that the final cell concentration is 50,000 cells per well. Make ten-fold dilutions of the viral stock. Add 50 µl to the test wells. Add 50 µl growth medium to bring the total test volume to 200 µl. On day 3 place a clear plastic film (plate sealers, Dynatech Laboratories, Inc., cat # 001-010-3501) over the microtiter wells and evaluate for syncytium forming units (SFU).

**A.5. Evaluation of antiviral activity of compounds with the syncytial- forming assay.**

The assay described above for determining viral titers of HIV-1 stock virus can be modified and used to evaluate the antiviral activity of compounds.

**A.5.1.** Plates are treated with poly-L-lysine the same as above, and CEM-SS cells are polybrene treated.

**A.5.2.** Dilute test compound in medium to desired concentrations.

**A.5.3.** Add 100 µl CEM-SS cells to each well so that the final cell concentration is 50,000 cells per well.

**A.5.4.** Add 50 µl of the test compound at each respective concentration to at least two wells, or four for one replicate assay.

**A.5.5.** Add 50 µl growth medium to drug control wells.

**A.5.6.** Add 50 µl HIV-1 virus to test wells at a concentration that provides approximately 100 SFU of virus per well.

**A.5.7.** Run appropriate cell and viral controls.

**A.5.8.** Incubate plates for 3 to 5 days.

**A.5.9.** On day 3 to 5 cover plates with clear plastic sealers and count syncytial-forming units in the test wells and viral control wells. To determine if the test compounds have antiviral activity, compare the total number of SFU in the viral control to those enumerated in the test wells.

**B. XTT Cytoprotection Assay for Screening Crude Extracts and Pure Compounds for Antiviral Activity against HIV-1**

This assay is a measure of the viability of human T cell lines after infection with HIV in the presence of potential antiviral compounds. The results are assessed spectrophotometrically by reading optical densities after the addition of XTT to determine if the viable cells reduced the pale yellow tetrazolium reagent (XTT) to an orange formazan product. This assay can be utilized for the initial screen of large numbers of samples.

The general protocol summarized for the XTT cytoprotection assay was provided by Dr. Owen Weislow. Changes were made to accommodate changes in solvent systems and drug test dilutions.

For the cytoprotection assay CEM-SS cells and HIV-1 RF strain of virus were used. Test samples were first diluted in an appropriate solvent, and 10 to 50 μl were transferred to the test plate. If the solvent was methanol or a similar solvent, the plates were allowed to evaporate. If medium was used for dilutions the plates were covered and the volume of test sample was taken into consideration when adding cells and virus. At each test concentration 50 μl of sample were added to three wells, one for toxicity tests, one for color control, and one for antiviral activity. Assuming the solvent was evaporated, 150 μl cells are added to all wells, except the control wells for medium only, so that the cell concentration per well was 5000. Then 50 μl of HIV-1 virus was added to each test well so that the M.O.I. was approximately 0.31. For toxicity tests 50 μl of medium were added to the control well. The plates were incubated in 5% $CO_2$ for 6 days at 37°C. After incubation, the XTT solution was added to the plates, and after 4 hours incubation at 37°C the plates are covered with clear plastic sealers. Cell viability was measured by the visible light absorbance at 450 nm and a reference wavelength of 650 nm. Data were expressed as a percentage of formazan produced in drug test wells compared to formazan produced in wells of untreated control cells.

**B.1. CEM-SS cells**

CEM-SS cells were maintained with growth medium RPMI 1640, 10% fetal bovine serum, 2% L-glutamine (200 mM), and 50 μg/ml gentamicin in a 5% $CO_2$ water-saturated atmosphere. Cells were maintained in the logarithmic growth state by subdividing cultures when the cell concentrations reach an upper limit of 1 x $10^6$ cells per ml.

For antiviral assays CEM-SS cells at a cell concentration of 5.0 x $10^5$ in serum free medium were treated with polybrene at a concentration of 1 μg/ml and incubated for 30 to 60 minutes at 37°C. Polybrene medium was removed by centrifugation, and CEM-SS cells were resuspended in growth medium. If solvents were evaporated the cell concentration was 33,500 cells per ml so that 150 μl contained 5000 cells. If medium was the dilution vehicle then the cell concentration was 50,000 so that 100 μl contained 5000 cells. The total test volume for medium, cells, and virus was 200 μl.

**B.2. Experimental Protocol for XTT Assay**

**B.2.1. Preparation of PMS stock**

PMS: Sigma: Cat# P 9625 - PMS, Phenazine methosulfate (N-Methyldibenzopyrazine methyl sulfate salt), yellow crystal

To prepare PMS stock weigh out 76.5 mg of PMS and solubilize in 5 ml of PBS. Store frozen at -20°C (0.1 to 0.2 ml per vial). Warm to 37°C as needed. Do not refreeze.

**B.2.2.** PMS dilution for XTT medium: Dilute PMS stock from freezer 1:100 in PBS. Add 4% to medium.

**B.2.3.** XTT - Source: Polysciences, Inc., Warrington, PA 18976-2590, Cat. #19661 (100 mg or 500 mg)

**B.3.4. Formula for XTT/PMS solution for 1 plate (5 ml per plate)**

| Component | Amount | Final Concentration |
|---|---|---|
| XTT | 5 mg | 1 mg/ml |
| PMS/PBS | 0.2 ml | 4.0 % (v/v) (.02M) |
| RPMI without phenol red | 4.8 ml | |

Dissolve XTT in medium, add PMS/PBS. Warm to 37°C for approximately 10 to 15 minutes to obtain an homogeneous solution. Add 50 µl per plate Incubate for 4 hours. Read A(450-650) nm.

**B.2.5. Data processing**

After the plates were read spectrophotometrically the data was transferred via modem from the laboratory to the office. The data was processed to convert optical density readings to a percentage of inhibition compared to CEM-SS cells that have not been infected with HIV-1 virus.

C = cells
D = Drug or Sample
M = Medium
V = Virus

Description of test wells:

M = medium only in well
V = medium + CEM-SS cells + virus in well
(M + C) = medium + CEM-SS cells
(M + D) = medium + drug in well
(M + D + C) = medium + drug + CEM-SS cell in well
(M+D+C+V) = medium + drug + CEM-SS cells + virus in well.

Calculations:

$$\% \text{ toxicity} = 100 - [(((M + D + C) - M) \div ((M + C) - M)) * 100]$$
$$\% \text{ antiviral} = ((M + D + C + V)-V-M)) \div ((M + D + C)-(V-M))) * 100$$

**C. Secondary HIV-1 Assays**

Secondary assays for evaluating antiviral activity included the syncytial-forming assay and p24 ELISA's. The first assay was described above. The p24 assay was performed by purchasing the p24 kit for the human immunodeficiency virus for p24 antigen assays from the Coulter Corporation, Hialeah, FL. A 24 kilodalton protein (p24) which is immunologically distinct from proteins in most other retroviruses, has been demonstrated to be a major structural core component of HIV-1 (Epstein et al., "The Molecular Biology of Human Immunodeficiency Virus Type 1 Infection," *New England J. Med.*, **324**: 308 (1991)). The preparation of a mouse monoclonal antibody with high specificity and affinity for the viral p24 protein has allowed the development of an extremely sensitive ELISA for HIV-1 p24 core antigen.

**D. Activity Studies**

The following Table illustrates the antiviral activity of the racemic mixture PM-92131(±) against HIV.

**Table I: HIV-1 Antiviral Activity of the Racemic Mixture PM-92131 (±)**

| Assay | $EC_{50}$ µg/200 µl | $IC_{50}$ µg/200 µl | SI |
|---|---|---|---|
| Cytotoxic Assay | >0.01 * | 1.0 | >100 |
| Cytotoxic Assay | 0.07 ** | 2.5 | 36 |
| P24 ELISA | 0.1 | | |
| Syncytia assay | 0.05 *** | | |

* Test date: 10-28-92
** Test date: 4-2-93 (Sample was a new synthesis)
*** M.O.I. of .025

A sample labeled "PM-92131 (±)," a racemic mixture of PM-92131(+) and PM-92131(-) was received at PharmaMar, USA, Cambridge, MA, from PharmaMar, Madrid, Spain on 10-23-92. An assay for antiviral activity to HIV-1 was started on 10-28-93 using the cytotoxic assay as described above and HIV-1 activity was observed microscopically in this sample on day 4 of the cytotoxic assay. On day six, 10 µl of supernatant fluid were removed from the cytotoxic wells and assayed for p24. The results of the cytotoxic assay, p24 assay, and syncytia assay were:

**Table II. HIV-1 Antiviral Activity of PM-92131 (±)**

| Assay | Assay Date | $EC_{50}$ µg/200 µl | $IC_{50}$ µg/200 µl | SI |
|---|---|---|---|---|
| *PM-92131 (±) (253-48) | 10-28-92 | >0.01 | 1.0 | >100 |
| **PM-92131 (±) (253-48) | 10-28-92 | >0.01 | | |
| *PM-92131 (±) (253-48) | 11-11-92 | 0.05 | 1 | 20 |
| ***PM-92131 (±) (253-48) | 11-11-92 | 0.05 | 0.91 | 18 |
| *PM-92131 (±) (253-48) | 12-11-92 | 0.15 | 5 | 33 |
| **PM-92131 (±) (253-48) | 12-11-92 | 0.25 | | |
| *PM-92131 (±) (289-8) | 04-02-93 | 2 | >10 | >5 |
| ***PM-92131 (±) (289-8) | 04-02-93 | 0.12 | >5 | >42 |

* Cytotoxic Assay
** p24 ELISA Assay
*** Syncytia Assay

PM-92131 (±) was confirmed in subsequent assays to be a volatile substance (camphor-like), as it was found that the activity of the compound was not confined to the respective test wells. HIV-1 virus in test wells adjacent to the wells containing the compound was inactivated by the motility of the volatile substance in the adjacent well(s) .

**Toxicity Test**

Approximately 16.7 mg of PM-92131 (±) (289-8) was received at PharmaMar USA on 12/16/92 for *in vivo* cytotoxic tests. The recipient CD-1 mice survived the highest test concentration with a mean survival time greater than five days after the single injection. The $LD_{50}$ for the compound was > 150 mg/kg. This sample had HIV-1 antiviral activity (see data for 289-8) in Table 1.

**E. Synthetic Test Samples**

PharmaMar USA received additional quantities of newly synthesized racemic PM-92131 (±) (322-5) on 3/4/93 and the two separated enantiomers on 4/5/93 (PM-92131 (-) and PM-92131 (+). All three of the newly synthesized compounds were active against HIV-1, but one of the enantiomers (PM-92131 (+)) was significantly more active than the other enantiomer (PM-92131 (-)). The HIV-1 antiviral *in vitro* assays and results for these 3 samples are given in Table III.

**Table III. HIV-1 Antiviral Activity of PM-92131 (±), PM-92131 (-) and PM-92131 (+)**

| Assay | Assay Date | $EC_{50}$ µg/200 µl | $IC_{50}$ µg/200 µl | SI |
|---|---|---|---|---|
| *PM-92131 (±) (322-5) | 04-02-93 | 0.15 | 2.25 | 15 |
| ***PM-92131 (±) (322-5) | 04-02-93 | 0.07 | | |
| *PM-92131 (±) (289-8) | 04-02-93 | 2 | >10 | >5 |
| ***PM-92131 (±) (289-8) | 04-02-93 | 0.12 | >5 | >42 |
| *PM-92131 (±) (322-5) | 04-27-93 | 0.4 | 2.2 | 6 |
| **PM-92131 (±) (322-5) | 04-27-93 | 0.07 | | |
| ***PM-92131 (±) (253-48) | 04-27-93 | 0.12 | 2.5 | 21 |
| *PM-92131 (+) (333-38) | 04-27-93 | 0.18 | 1.2 | 7 |
| **PM-92131 (+) (333-3) | 04-27-93 | 0.15 | | |
| ***PM-92131 (+) (333-3) | 04-27-93 | 0.08 | 7 | 88 |
| *PM-92131 (-) (333-2) | 04-27-93 | No activity | | |
| ***PM-92131 (-) (333-2) | 04-27-93 | 0.8 | 8 | 10 |

* Cytotoxic Assay

** p24 ELISA Assay

*** Syncytia Assay (M.O.I. = .005)

The activity of PM-92131 (±) was compared to AZT. The first comparison was made testing both compounds for HIV-1 antiviral activity enumerating SFU's. Several observations were made.

Table IV.

| Antiviral Activity of PM-92131 (±) vs. AZT in Syncytia Assay | | |
|---|---|---|
| | $EC_{50}$ (μM) | |
| MOI | PM-92131 (±) | AZT |
| 0.016 | 2.30 | 0.07 |
| 0.008 | 1.20 | 0.01 |
| 0.004 | 0.58 | 0.01 |
| 0.002 | 0.58 | 0.01 |

At higher doses of input virus, the HIV-1 antiviral activity of PM-92131 (±) decreased less dramatically than AZT.

A second comparison was made with the data from the cytoprotection assay:

Table V.

| Antiviral Activity of PM-92131 (±) vs. AZT in the Cytoprotection Assay | | |
|---|---|---|
| | $EC_{50}$ (μM) | |
| MOI | PM-92131(±) | AZT |
| 0.149 | 1.70 | 1.30 |
| 0.074 | 1.20 | 0.46 |
| 0.037 | 1.30 | 0.10 |

At low viral concentrations, AZT is a better inhibitor of viral replication. However, as the concentration of test virus is increased, the activity of PM-92131 (±) comes closer to AZT. This indicates that PM-92131 (±) could be as effective as AZT for inhibiting HIV-1.

The antiviral activities of some of the new synthetic compounds disclosed herein are shown in the next table.

**Table VI. HIV-1 Antiviral Activity of Several New Compounds**

| Assay | Assay Date | $EC_{50}$ µg/200 µl | $IC_{50}$ µg/200 µl | SI |
|---|---|---|---|---|
| SU-0096(±) | 06-29-94* | 0.15 | 0.90 | 6 |
| | 09-06-93* | 0.10 | 1.12 | 11 |
| | 09-06-93* | 0.21 | 1.00 | 5 |
| PM-94118(±) | 06-29-94* | 0.60 | 1.80 | 3 |
| | 09-06-93* | 0.47 | 1.87 | 1 |
| | 09-06-93* | 0.94 | 1.87 | 2 |
| | 06-29-94** | 0.40 | 4.00 | 10 |
| PM-94116 & | 06-29-94* | 0.60 | 0.90 | 2 |
| PM-94117 | 10-29-93* | 0.43 | 0.87 | 2 |
| (1:1) | 06-29-94** | 0.20 | 2.20 | 11 |
| PM-94116 | 06-29-94* | 0.30 | 0.60 | 2 |
| | 04-15-94* | 0.08 | 0.94 | 12 |
| | 06-29-94** | 0.07 | 1.90 | 35 |
| PM-94117 | 06-29-94* | 2.50 | 2.50 | 1 |
| | 04-15-94* | 0.94 | 2.50 | 3 |
| | 06-29-94** | 0.24 | 3.00 | 13 |

* Cytotoxic Assay
** Syncytia Assay

PM-92131 (±), its separated enantiomers, and all of the synthetic samples tested to date, have volatile properties. The volatile substance inactivates HIV-1 in adjacent wells that contain virus, cells and medium and no test drug. Toxicity is observed in wells with cells and medium that are adjacent to wells containing the test drug at toxic doses. This specific property of these compounds makes it difficult to obtain accurate values of the *in vitro* HIV-1 antiviral activity using this test methodology. Thus, the most accurate data for the evaluation of these compounds for HIV-1 *in vitro* HIV-1 antiviral activity was the syncytium forming assay.

The racemic mixture PM-92131 (±), and both separated enantiomers exhibit anti-HIV-1 activity, but the enantiomeric form, PM-92131 (+) appears to be the most active compound. The racemic mixture PM-94116 & PM-94117 and both separated enantiomers exhibit anti-HIV-1 activity, but the enantiomeric form, PM-94116, appears to be the most active

anti-HIV-1 compound.

The present invention has been described in detail, including the preferred embodiments thereof. However, it will be appreciated that those skilled in the art, upon consideration of the present disclosure, may make modifications and/or improvements on this invention and still be within the scope and spirit of this invention as set forth in the following claims.

## Claims

1. Substituted 6,7-epoxy-bicyclo-[4.3.0]-nonan-8-ones having the general formula:

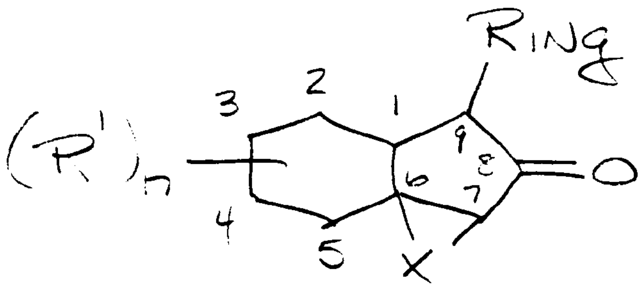

wherein X is selected from the group consisting of O and S; each of the n = 1-9 substituent groups $R^1$ are independently selected from the group consisting of hydrogen, halogen, hydroxy, $CF_3$, $C_1$-$C_6$ acyl, $C_1$-$C_6$ alkyl, $C_2$-$C_6$ alkenyl, $C_2$-$C_6$ alkynyl, $C_1$-$C_6$ alkoxy, $C_6$-$C_{18}$ aryl, $C_2$-$C_6$ dialkoxymethyl, cyano, $C_3$-$C_6$ cycloalkyl, $C_3$-$C_6$ heterocycloalkyl, $C_3$-$C_{15}$ dialkylaminoalkyl, carboxy, $C_2$-$C_6$ carboxylic acid, carboxamido, $C_1$-$C_6$ haloalkyl, $C_1$-$C_6$ haloalkylthio, allyl, $C_7$-$C_{20}$ aralkyl, a $C_3$-$C_6$ heterocycloalkyl ring fused to a benzene ring, $C_1$-$C_6$ alkylthio, $C_1$-$C_6$ alkylsulfonyl, $C_1$-$C_6$ haloalkylsulfonyl, $C_1$-$C_6$ alkylsulfinyl, $C_1$-$C_6$ haloalkylsulfinyl, arylthio, $C_1$-$C_6$ haloalkoxy, amino, $C_1$-$C_6$ alkylamino, $C_2$-$C_{15}$ dialkylamino, carbamoyl, $C_1$-$C_6$ N-alkyl-carbamoyl, $C_2$-$C_{15}$ N,N-dialkylcarbamoyl, nitro, and $C_2$-$C_{15}$ dialkylsulfamoyl;
and wherein a substituted or unsubstituted $C_5$-$C_8$ carbocyclic or $C_3$-$C_5$ heterocyclic ring is located at position 9, the substituents being selected from those defined above for $R^1$;
with the proviso that when X is oxygen - $R^1$ at position 1 is not methyl; or $R^1$ at position 5 is not dimethyl; or the ring at position 9 is not unsubstituted furan.

2. Substituted bicyclo-[4.3.0]-nonan-8-one compounds of claim 1, having the following formula:

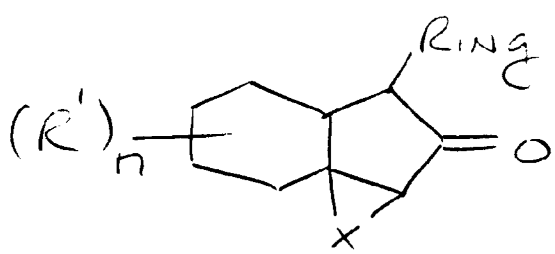

wherein n is an integer from 1 to 5, and each $R^1$ is independently selected from H and lower alkyl.

3. Substituted bicyclo-[4.3.0]-nonan-8-ones of claim 2, wherein X is oxygen, n is 1, 2, or 3 and each $R^1$ is independently a lower alkyl group.

4. Substituted bicyclo-[4.3.0]-nonan-8-one compounds of claim 2, wherein X is sulfur, n is 1, 2, or 3 and each $R^1$ is independently a lower alkyl group.

5. The compounds of claim 1, 2, 3 or 4, wherein the carbocyclic or heterocyclic ring substituent at position 9 is selected from the group consisting of substituted and unsubstituted rings including cyclopentadiene, pyrrole, furan, thiophene, benzene, pyridine, pyran, pyrone, naphthalene, quinoline, isoquinoline, quinolizine, acridine, phenanthridine, benzopyran, pyrazole, imidazole, isoxazole, oxazole, isothiazole, thiazole, pyridazine, pyrimidine, pyrazine, oxazine, thiazine, dioxane, purine, pteridine, triazine, sydnone, borepine, azepine, ozepine and thiepin; said substituents being selected from those defined for $R^1$.

6. Substituted bicyclo-[4.3.0]-nonan-8-ones of claim 5, wherein the carbocyclic or heterocyclic ring substituent at position 9 is selected from the group consisting of:

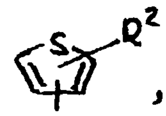, 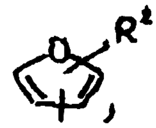, 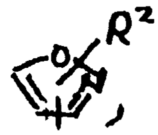,

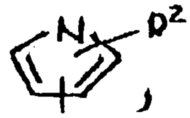, 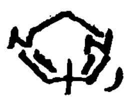, 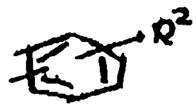

wherein $R^2$ is defined the same as $R^1$.

**7.** Substituted bicyclo-[4.3.0]-nonan-8-ones of claim 5, selected from the group consisting of:

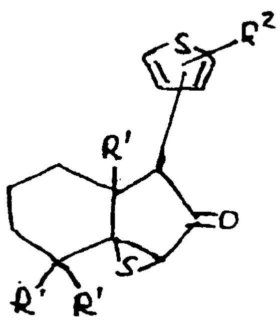 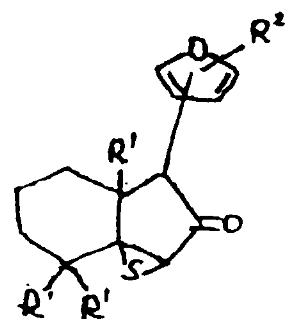 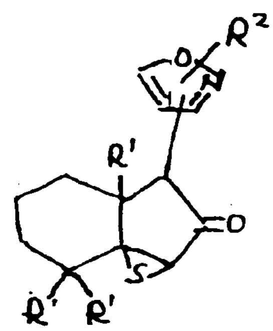

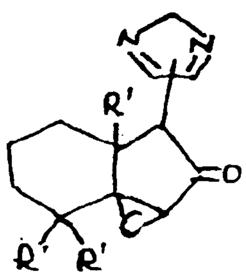 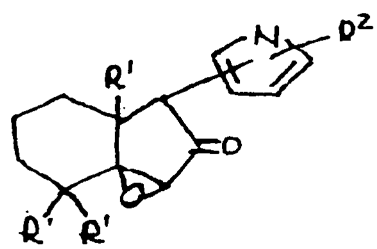 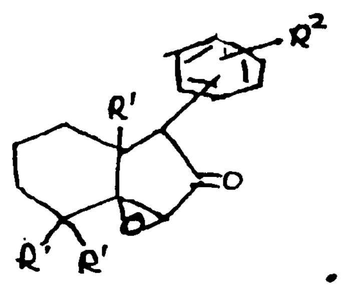.

**8.** Substituted bicyclo-[4.3.0]-nonan-8-ones of claim 5, selected from the group consisting of:

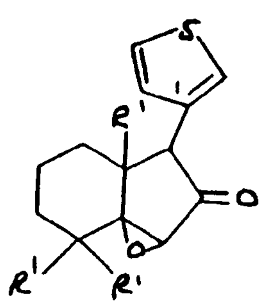 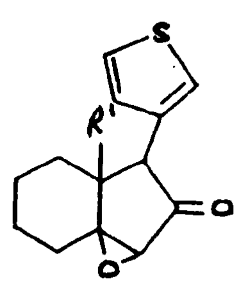 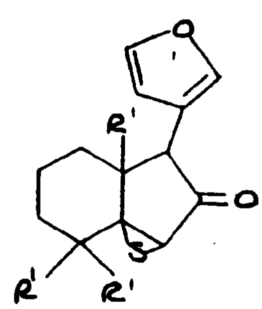

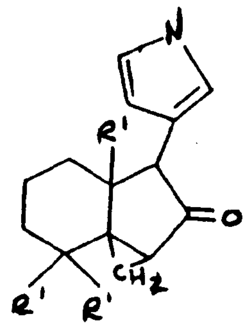

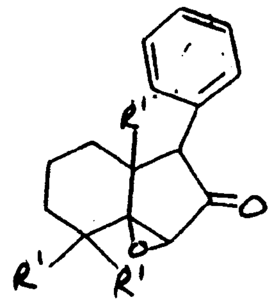

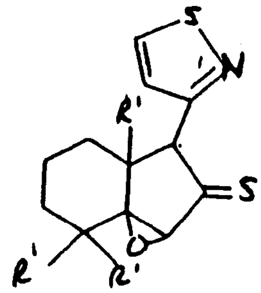

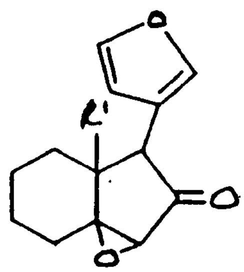

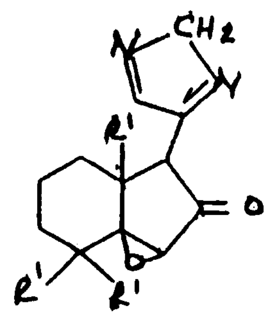

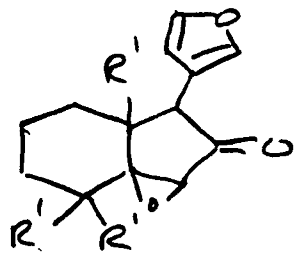

**9.** Substituted bicyclo-[4.3.0]-nonan-8-ones of claim 3, wherein Ring is selected from the group consisting of substituted and unsubstituted phenyl and thiophene.

**10.** Substituted bicyclo-[4.3.0]-nonan-8-ones of claim 4, wherein Ring is selected from the group consisting of substituted and unsubstituted phenyl and thiophene.

**11.** The substituted bicyclo-[4.3.0]-nonan-8-one compound of claim 3, having the following specific configuration:

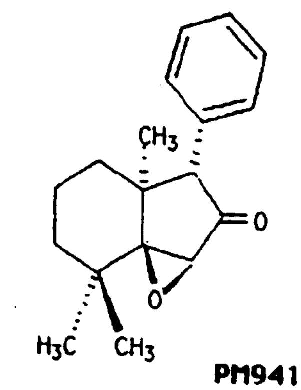

**PM94118(±)**

**12.** The substituted bicyclo-[4.3.0]-nonan-8-one compound of claim 3, having the following specific configuration:

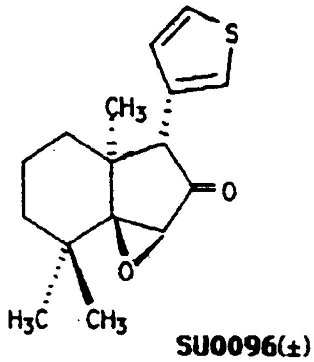

SU0096(±)

**13.** The substituted bicyclo-[4.3.0]-nonan-8-one compound of claim 3, having the following specific configuration:

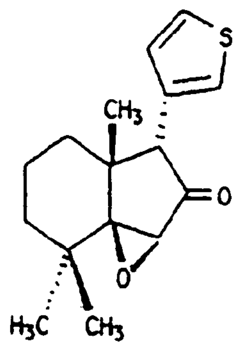

PM94116

**14.** The substituted bicyclo-[4.3.0]-nonan-8-one compound of claim 3, having the following specific configuration:

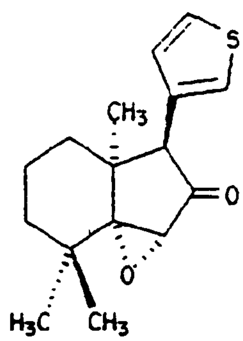

PM94117

**15.** The 1:1 racemic mixture of substituted bicyclo-[4.3.0]-nonan-8-one compounds having the following specific configuration:

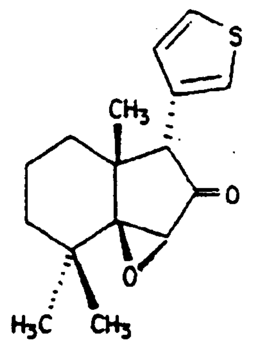

PM94116+PM94117

**16.** Compounds having the formula:

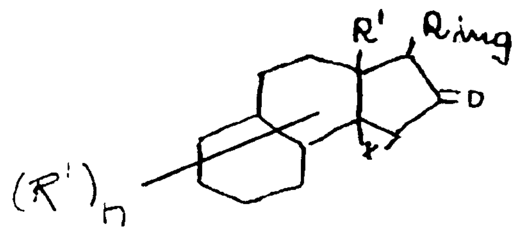

wherein X is selected from the group consisting of O and S; each of the n = 1-9 substituent groups $R^1$ are independently selected from the group consisting of hydrogen, halogen, hydroxy, $CF_3$, $C_1$-$C_6$ acyl, $C_1$-$C_6$ alkyl, $C_2$-$C_6$ alkenyl, $C_2$-$C_6$ alkynyl, $C_1$-$C_6$ alkoxy, $C_6$-$C_{18}$ aryl, $C_2$-$C_6$ dialkoxymethyl, cyano, $C_3$-$C_6$ cycloalkyl, $C_3$-$C_6$ heterocycloalkyl, $C_3$-$C_{15}$ dialkylaminoalkyl, carboxy, $C_2$-$C_6$ carboxylic acid, carboxamido, $C_1$-$C_6$ haloalkyl, $C_1$-$C_6$ haloalkylthio, allyl, $C_7$-$C_{20}$ aralkyl, a $C_3$-$C_6$ heterocycloalkyl ring fused to a benzene ring, $C_1$-$C_6$ alkylthio, $C_1$-$C_6$ alkylsulfonyl, $C_1$-$C_6$ haloalkylsulfonyl, $C_1$-$C_6$ alkylsulfinyl, $C_1$-$C_6$ haloalkylsulfinyl, arylthio, $C_1$-$C_6$ haloalkoxy, amino, $C_1$-$C_6$ alkylamino, $C_2$-$C_{15}$ dialkylamino, carbamoyl, $C_1$-$C_6$ N-alkyl-carbamoyl, $C_2$-$C_{15}$ N,N-dialkylcarbamoyl, nitro, and $C_2$-$C_{15}$ dialkylsulfamoyl;
and wherein a substituted or unsubstituted $C_5$-$C_8$ carbocyclic or $C_3$-$C_5$ heterocyclic ring is located at position 9, the substituents being selected from those defined above for $R^1$ above.

**17.** Compounds having the formula:

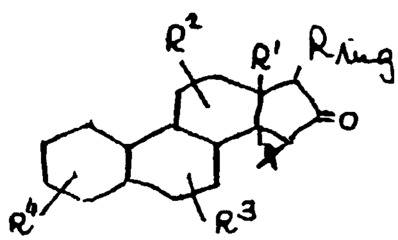

wherein X is selected from the group consisting of O and S; each of the n = 1-9 substituent groups $R^1$, $R^2$, $R^3$ and $R^4$ are independently selected from the group consisting of hydrogen, halogen, hydroxy, $CF_3$, $C_1$-$C_6$ acyl, $C_1$-$C_6$ alkyl, $C_2$-$C_6$ alkenyl, $C_2$-$C_6$ alkynyl, $C_1$-$C_6$ alkoxy, $C_6$-$C_{18}$ aryl, $C_2$-$C_6$ dialkoxymethyl, cyano, $C_3$-$C_6$ cycloalkyl, $C_3$-$C_6$ heterocycloalkyl, $C_3$-$C_{15}$ dialkylaminoalkyl, carboxy, $C_2$-$C_6$ carboxylic acid, carboxamido, $C_1$-$C_6$ haloalkyl, $C_1$-$C_6$ haloalkylthio, allyl, $C_7$-$C_{20}$ aralkyl, a $C_3$-$C_6$ heterocycloalkyl ring fused to a benzene ring, $C_1$-$C_6$ alkylthio, $C_1$-$C_6$ alkylsulfonyl, $C_1$-$C_6$ haloalkylsulfonyl, $C_1$-$C_6$ alkylsulfinyl, $C_1$-$C_6$ haloalkylsulfinyl, arylthio, $C_1$-$C_6$ haloalkoxy, amino, $C_1$-$C_6$ alkyamino, $C_2$-$C_{15}$ dialkylamino, carbamoyl, $C_1$-$C_6$ N-alkyl-carbamoyl, $C_2$-$C_{15}$ N,N-dialkylcarbamoyl, nitro, and $C_2$-$C_{15}$ dialkylsulfamoyl;
and wherein a substituted or unsubstituted $C_5$-$C_8$ carbocyclic or $C_3$-$C_5$ heterocyclic ring is located at position 9, the substituents being selected from those defined above for $R^1$ above.

**18.** Pharmaceutical compositions comprising a pharmaceutically acceptable carrier, diluent or excipient, and an effective antiviral amount, particularly an effective anti-retroviral amount of a substituted bicyclo-[4.3.0]-nonan-8-one compound having the following generic formula:

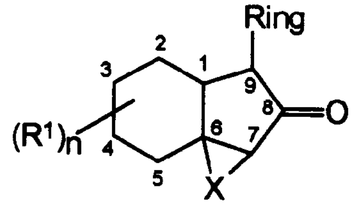

wherein X is selected from the group consisting of O and S; n is an integer of 1-9; each $R^1$ is independently H or a $C_1$ to $C_4$ lower alkyl group; and Ring is a $C_3$ to $C_6$ carbocylic or $C_2$ to $C_5$ heterocyclic ring system with up to three heteroatoms selected from nitrogen, sulfur and/or oxygen, and wherein either ring system may containing up to three double bonds.

**19.** The pharmaceutical composition of claim 18, wherein the carboxylic or heterocyclic ring system at position 9 of the active compounds defined therein are selected from the group consisting of substituted and unsubstituted rings including cyclopentadiene, pyrrole, furan, thiophene, benzene, pyridine, pyran, pyrone, naphthalene, quinoline, isoquinoline, quinolizine, acridine, phenanthridine, benzopyran, pyrazole, imidazole, isoxazole, oxazole, isothiazole, thiazole, pyridazine, pyrimidine, pyrazine, oxazine, thiazine, dioxane, purine, pteridine, triazine, sydnone, borepine, azepine, ozepine and thiepin.

**20.** The pharmaceutical composition of claim 19, wherein the carbocyclic or heterocyclic ring substituent at position 9 is selected from the group consisting of:

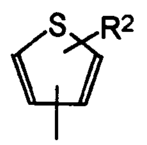 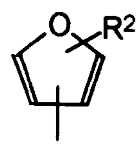 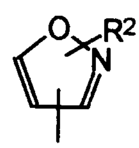

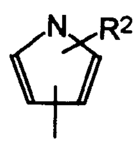 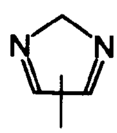 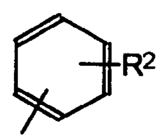

wherein $R^2$ is defined the same as $R^1$.

**21.** The pharmaceutical composition of claim 18, wherein the active ingredient is a substituted bicyclo-[4.3.0] -nonan-8-one selected from the group consisting of:

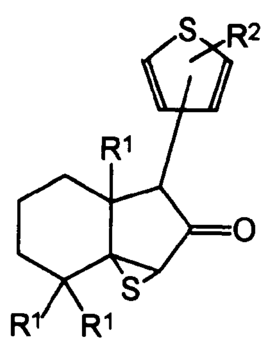 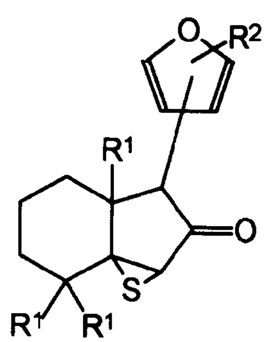 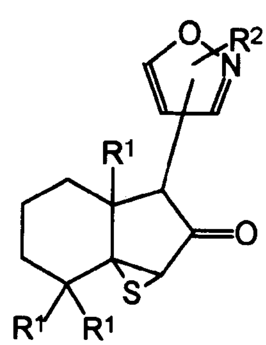

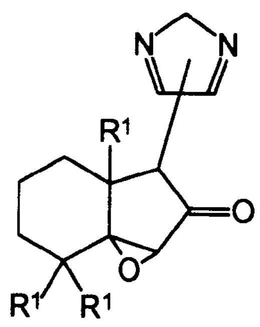
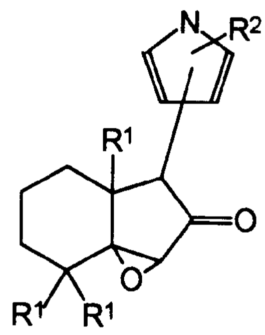
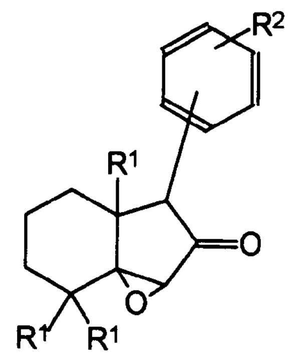

wherein $R^1$ and $R^2$ are as defined above.

**22.** The pharmaceutical composition of claim 18, wherein the active ingredient is a substituted bicyclo-[4.3.0] -nonan-8-one selected from the group consisting of:

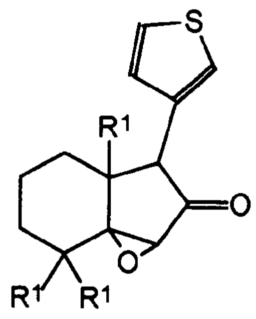
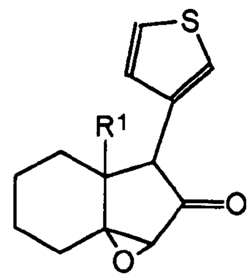
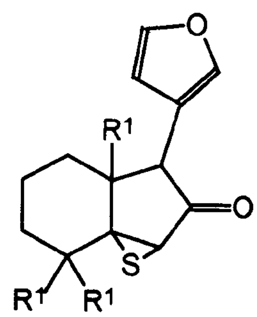
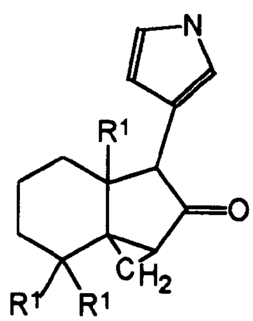
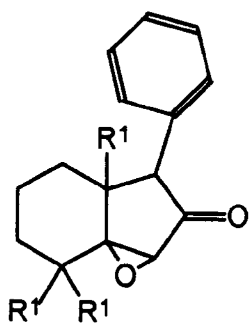
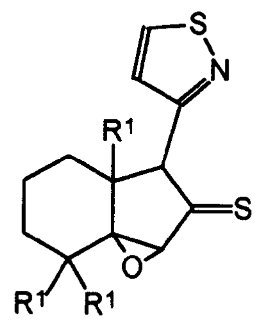
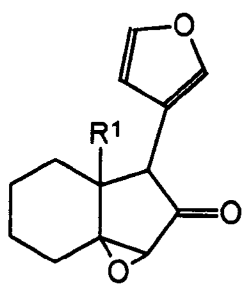
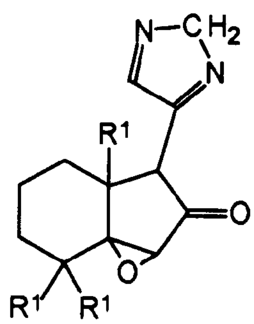
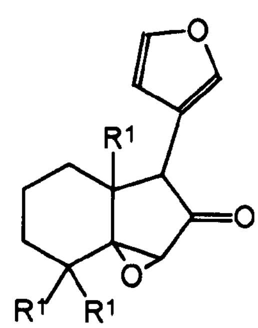

**23.** The pharmaceutical composition of claim 18, wherein the active ingredient is a substituted bicyclo-[4.3.0] -nonan-8-one, wherein X is oxygen and Ring is selected from the group consisting of substituted and unsubstituted phenyl and thiophene.

**24.** The pharmaceutical composition of claim 18, wherein the active ingredient is a substituted bicyclo-[4.3.0] -nonan-8-one, wherein X is sulfur and Ring is selected from the group consisting of substituted and unsubstituted phenyl and thiophene.

**25.** The pharmaceutical composition of claim 23, wherein the active ingredient is a substituted bicyclo-[4.3.0] -nonan-8-one, having the following specific configuration:

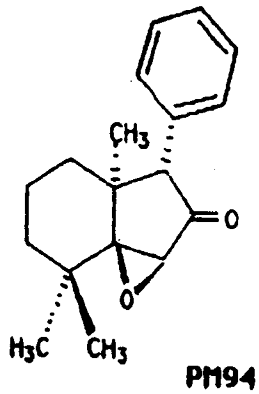

**PM94118(±)**

**26.** The pharmaceutical composition of claim 23, wherein the active ingredient is a substituted bicyclo-[4.3.0] -nonan-8-one, having the following specific configuration:

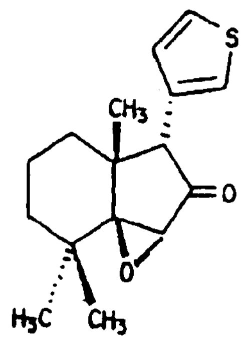

**PM94116**

**27.** The pharmaceutical composition of claim 23, wherein the active ingredient is a substituted bicyclo-[4.3.0] -nonan-8-one, having the following specific configuration:

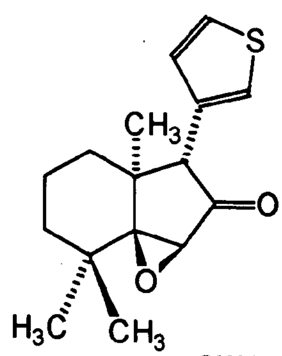

**SU0096(±)**

**28.** The pharmaceutical composition of claim 23, wherein the active ingredient is a substituted bicyclo-[4.3.0] -nonan-8-one, having the following specific configuration:

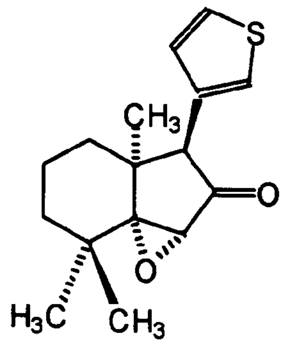

**PM94117**

29. The pharmaceutical composition of claim 23, wherein the active ingredient is the 1:1 racemic mixture of substituted bicyclo-[4.3.0]-nonan-8-one compounds having the following specific configuration:

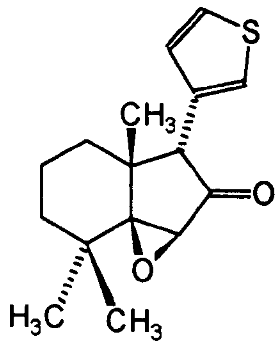

**PM94116+PM94117**

30. A method of treatment of viral infections, comprising administering to a patient in need of such treatment, an effective antiviral amount of a substituted bicyclo-[4.3.0]-nonan-8-one compound having the formula:

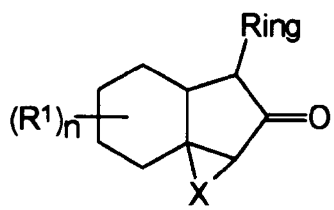

wherein Z is selected from the group consisting of O and S; each of the n = 1-9 substituent groups $R^1$ are independently selected from the group consisting of hydrogen, halogen, hydroxy, $CF_3$, $C_1$-$C_6$ acyl, $C_1$-$C_6$ alkyl, $C_2$-$C_6$ alkenyl, $C_2$_$C_6$ alkynyl, $C_1$-$C_6$ alkoxy, $C_6$-$C_{18}$ aryl, $C_2$-$C_6$ dialkoxymethyl, cyano, $C_3$-$C_6$ cycloalkyl, $C_3$-$C_6$ heterocycloalkyl, $C_3$-$C_{15}$ dialkylaminoalkyl, carboxy, $C_2$-$C_6$ carboxylic acid, carboxamido, $C_1$-$C_6$ haloalkyl, $C_1$-$C_6$ haloalkylthio, allyl, $C_7$-$C_{20}$ aralkyl, a $C_3$-$C_6$ heterocycloalkyl ring fused to a benzene ring, $C_1$-$C_6$ alkylthio, $C_1$-$C_6$ alkylsulfonyl, $C_1$-$C_6$ haloalkylsulfonyl, $C_1$-$C_6$ alkylsulfinyl, $C_1$-$C_6$ haloalkylsulfinyl, arylthio, $C_1$-$C_6$ haloalkoxy, amino, $C_1$-$C_6$ alkylamino, $C_2$-$C_{15}$ dialkylamino, carbamoyl, $C_1$-$C_6$ N-alkyl-carbamoyl, $C_2$-$C_{15}$ N,N-dialkylcarbamoyl, nitro, and $C_2$-$C_{15}$ dialkylsulfamoyl;
and wherein a substituted or unsubstituted $C_5$-$C_8$ carbocyclic or $C_3$-$C_5$ heterocyclic ring is located at position 9, the substituents being selected from those defined above for $R^1$ above.

31. The method of claim 30, wherein the viral infection is a retroviral infection.

32. The method of claim 31, wherein the retroviral infection is a human retroviral infection.

33. The method of claim 32, wherein the human retroviral infection is selected from the group consisting of HIV-I, HIV-2,

HTLV-I and HTLV-II infections.

**34.** The method of claim 31, wherein the retroviral infection is an animal retroviral infection.

**35.** The method of claim 34, wherein the animal retroviral infection is selected from the group consisting of disease states caused by the feline immunodeficiency virus and the feline leukemia virus.

**36.** The method of claim 30, wherein the active ingredient is a substituted bicyclo-[4.3.0]-nonan-8-one, having the following specific configuration:

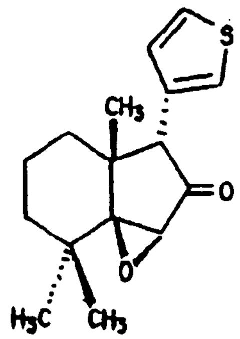

**37.** The method of claim 30, wherein the active ingredient is a substituted bicyclo-[4.3.0]-nonan-8-one, having the following specific configuration:

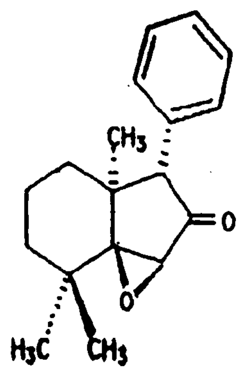

**38.** The method of claim 30, wherein the active ingredient is a substituted bicyclo-[4.3.0]-nonan-8-one, having the following specific configuration:

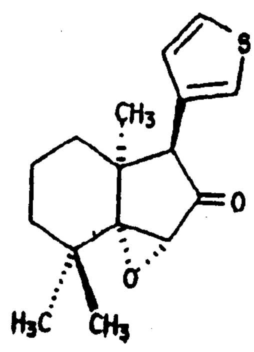

**39.** The method of claim 30, wherein the active ingredient is a substituted bicyclo-[4.3.0]-nonan-8-one, having the following specific configuration:

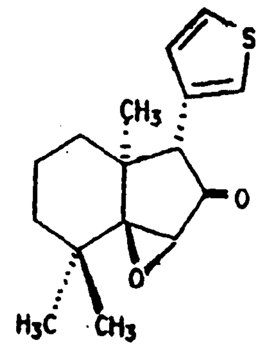

**40.** The method of claim 30, wherein the active ingredient is the 1:1 racemic mixture of substituted bicyclo-[4.3.0] -nonan-8-one compounds having the following specific configuration:

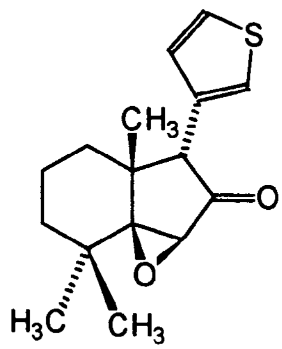

**41.** A method of treating immunodeficiency virus infections in animals and humans, comprising administering an antiviral effective amount of a compound having the following formula, the geometric and optical isomers thereof, and mixtures of those isomers, to an animal or human patient in need of such treatment:

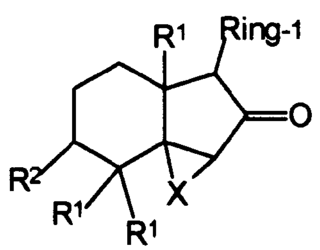

wherein X is selected from the group consisting of O and S; each $R^1$ is independently a $C_1$ to $C_4$ lower alkyl group; Ring-1 is a $C_2$-$C_6$ ring system containing up to three double bonds, and up to three heteroatoms selected from nitrogen, sulfur and/or oxygen; and $R^2$ is selected from H, O-glucose, or it serves, together with an adjacent carbon atom, as the attachment site for Ring-2, a $C_4$ to $C_{12}$ ring system.

**42.** A method of treating human immunodeficiency virus infections in animals and humans, comprising administering an antiviral effective amount of a compound having the following formula, the geometric and optical isomers thereof, and mixtures of those isomers and a pharmaceutically acceptable carrier, diluent or excipient:

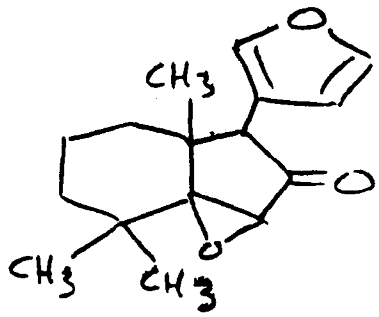

(I)

**43.** The method of claim 42, wherein the antiviral compound has the following relative configuration:

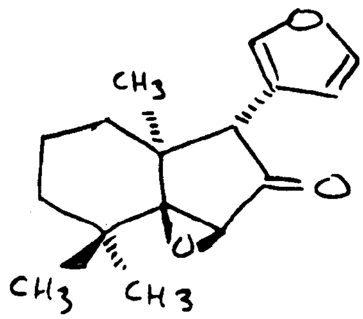

**44.** The method of claim 42, wherein the antiviral compound has the following relative configuration: